# EUROPÄISCHE PATENTSCHRIFT

(11) **EP 2 461 847 B1**
(45) Veröffentlichungstag und Bekanntmachung des Hinweises auf die Patenterteilung: **27.05.2015**
(21) Anmeldenummer: 10754677.2
(22) Anmeldetag: 09.08.2010
(51) Int. Cl.: A61M 1/36, A61M 1/16, A61M 1/34, B01D 53/22, B01D 63/02

(54) **VORRICHTUNG ZUR ELIMINIERUNG VON BIOSCHÄDLICHEN STOFFEN AUS KÖRPERFLÜSSIGKEITEN**
DEVICE FOR ELIMINATING BIOLOGICALLY HARMFUL SUBSTANCES FROM BODILY FLUIDS
DISPOSITIF POUR ÉLIMINER DES MATIÈRES BIOLOGIQUEMENT DÉLÉTÈRES CONTENUES DANS LES FLUIDES ORGANIQUES

(30) Priorität: 07.08.2009 DE 102009037015
(43) Veröffentlichungstag der Anmeldung: 13.06.2012
(73) Patentinhaber: Hemoteq AG, 52146 Würselen (DE)
(72) Erfinder: OTTO, Veit, 12587 Berlin (DE); HAJEK, Michaela, 12587 Berlin (DE)
(74) Vertreter: Arth, Hans-Lothar
(86) Internationale Anmeldenummer: PCT/DE2010/000954
(87) Internationale Veröffentlichungsnummer: WO 2011/015197

(56) Entgegenhaltungen:
- US-A1- 2004 228 829
- US-A1- 2005 182 349
- US-A1- 2008 138 434

## Beschreibung

Die vorliegende Erfindung betrifft eine Vorrichtung zur Reinigung von Blut, Blutersatzmitteln oder Lösungen zum Einbringen in den menschlichen und/oder tierischen Blutkreislauf und zum Gasaustausch in Blut, Blutersatzmitteln oder Lösungen zum Einbringen in den menschlichen und/oder tierischen Blutkreislauf, die mindestens eine gasdurchlässige Membran und einen Träger umfasst, der mit Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metabolite und Abbauprodukte beschichtet ist.

Die Entfernung von gesundheitsschädlichen Stoffen aus Blut wird schon seit langer Zeit praktiziert. Hierbei sind an erster Stelle die Dialyseverfahren zu nennen, die bei akuter und chronischer Niereninsuffizienz angewendet werden. Die Weiterentwicklung dieser Verfahren seit der Erstanwendung 1924 führte zu einer weltweit anerkannten und erfolgreich praktizierten Lebensrettungs- bzw. Lebensverlängerungsmaßnahme für Menschen mit Niereninsuffizienz. Auf dem Gebiet der Dialyse lässt sich vor allem der seit über 20 Jahren erfolgreich eingesetzte Hohlfaseradsorber der Fresenius AG nennen.

Seither haben diese extrakorporalen Verfahren in vielen Bereichen der Medizin Einzug genommen, in denen es gilt, Körperflüssigkeit von Schadstoffen zu befreien oder einen notwendigen Stoffaustausch vorzunehmen. Die hierzu genutzten Apparaturen sind in der Regel für eine spezielle Aufgabe entwickelt worden. Wie der Dialysator während der Hämodialyse das Plasma des Patienten von Abfallprodukten des Stoffwechsels als "künstlichen Niere" reinigt, so lassen sich auch Probleme der Immunabwehr mit Hilfe der Adsorber lösen. Nach Abtrennung der Blutzellen wird das Blutplasma über eine Apheresesäule geleitet, an der die krankmachenden Antikörper selektiv gebunden werden und das derart gereinigte Blutplasma dem Patienten wieder zugeführt. Für diese Fälle müssen die Säulen über die für diesen Antikörper notwendigen spezifischen Bindungsstellen verfügen, um die Antikörper binden zu können.

Obwohl die Möglichkeiten zunehmen, lebensbedrohliche Zustände durch die Entfernung der in Körperflüssigkeiten vorhandenen Ursachen zu verbessern, existiert bis heute noch ein hoher Bedarf an hämokompatiblen Materialien sowie schonend und effektiv funktionierenden Verfahren zur Entfernung von toxischen Stoffen aus Körperflüssigkeiten wie auch aus kontaminierten Lösungen, die dem Körper zugeführt werden sollen.

Gleichzeitig nimmt der Bedarf an Therapien zu, die sich mit Sekundärerkrankungen beschäftigen, da häufig nicht die Ersterkrankung, sondern die als Folge einer Erkrankung auftretenden Komplikationen zum Tode führen. Als bekanntes Beispiel lässt sich hier die Sepsis nennen, die zurzeit auf der Liste der häufigsten Todesursachen an 10. Stelle steht und deren Auftreten stetig steigt. Da 30% bis 50% der Patienten, die eine Sepsis erleiden, trotz Maximaltherapie auch an dieser sterben, stellt sie ein sehr ernst zu nehmendes Problem dar. Hinzu kommt eine sich verbreitende Widerstandsfähigkeit der Bakterien gegenüber Antibiotika, die in den Krankenhäusern bereits heute zu einem ebenfalls ernst zu nehmendem Problem heranwächst.

Hervorgerufen wird die Sepsis durch das Auftreten von Entzündungen, die allgemein nach Verletzungen und im Krankenhaus zumeist nach einem operativen Eingriff entstehen können. Ebenso spielen nosokiomale Infektionen eine immer noch wichtige Rolle im Klinikalltag. Beispielsweise sind katheterassoziierte Bakteriämien noch immer häufig auftretende Komplikationen.

Das durch die Entzündung aktivierte Immunsystem greift vorhandene gramnegative Bakterien (z.B. Escherichia coli, Pseudomonas aeruginosa, Enterobacter aerogenes, Salmonellen, Shigellen, Neisserien, wie die Meningokokken und der Erreger der Gonorrhoe) an. Es folgt die Lysierung der Bakterien und Abtransport der Abbauprodukte über die Blutbahn zu den Nieren. Zu diesen Abbauprodukten gehören auch Zellmembranbestandteile, die als Enterotoxine bzw. Endotoxine bzw. Lipopolysacharide (LPS) sich im gesamten Organismus verteilen und ihre toxische Wirkung entfalten können. Schafft die Immunabwehr des Körpers es nicht, den Entzündungsvorgang zu stoppen, gerät die Situation außer Kontrolle und die Infektion entwickelt sich zu einer Sepsis.

Als Standardtherapie gegen die Sepsis erhält der Patient in der Regel ein Antibiotikum, dessen Wirksamkeit auf die Bakterien im Vorfeld mikrobiologisch getestet worden ist. Wird bereits eine Organdysfunktion festgestellt, muss dieses Organ in seiner Funktion unterstützt (Organunterstützungstherapie) werden bzw. dieses Organ muss entsprechend vorübergehend therapeutisch ersetzt werden (Organersatztherapie). Atmung und Kreislauf müssen in diesem Stadium bereits stabilisiert werden. Greifen diese Maßnahmen nicht, kommt es zu weiteren Organausfällen und letztendlich zum Tod durch Multiorganversagen. Ein besonders ungünstiger Fall tritt zudem ein, wenn die notwendige Gabe von Antibiotika die Konzentration an Endotoxinen durch die Abtötung der Bakterien rasant steigen lassen, so dass die pathophysiologischen Vorgänge immens beschleunigt werden oder die Bakterien gegen die einsetzbaren Antibiotika widerstandsfähig sind und damit keine Standardtherapie mehr möglich ist.

Bis heute gibt es keine Adsorber, die erfolgreich die Sepsis-verursachenden Endotoxine aus dem Blut entfernen. Verschiedene Ansätze mit adsorbern zeigen bisher nicht den erhofften positiven Effekt.

Beispielsweise beschreibt DE 19648954A1 einen Endotoxinadsorber, der mit einem partikulären Träger arbeitet, an den kovalent Amin- bzw. Ammoniumgruppen aufweisende Liganden gekoppelt sind. DE 4113602A1 beschreibt einen Endotoxinadsorber mit perlförmigen Celluloseprodukten als Trägermaterial und Polyethylenimin als Ligand, während in DE 102006055558A1 das Trägermaterial aus einem Polysaccharid in beliebiger Form besteht und der aminogruppenhaltige Ligand bevorzugt Polyethylenimin oder Polyallylamin ist.

Eine Forschungsgruppe in München versuchte durch die Kopplung von L-Arginin an Träger einen Erfolg zu erzielen. Neben zwar vorteilhaften Nebeneffekten zeigte die Machbarkeitsstudie, die an 10 Patienten durchgeführt wurde, dass auch nach durchgeführter Plasmapherese die Endotoxinkonzentration immer noch unvermindert und damit erfolglos bei der Entfernung der Endotoxine war (Blood Purif. 2008; 26: 333-339). US2005/0182349 beschreibt eine Vorrichtung zur Reinigung von Blut umfassend einen Dialysator, einen Ultrafiltratgenerator, einen Oxygenator und einen Bioreaktor.

Somit besteht weiterhin ein hoher Bedarf an effektiven Lösungen für die Entfernung von gesundheitsgefährdenden Stoffen aus Blut, Blutersatzmitteln oder Lösungen zum Einbringen in den menschlichen und/oder tierischen Blutkreislauf, im speziellen für die Entfernung von Endotoxinen aus Vollblut und die effektive Behandlung von Sepsis.

Aufgabe der vorliegenden Erfindung ist es, eine Vorrichtung zur Verfügung zu stellen, welche effektiv Toxine biologischer und chemisch-synthetischer Herkunft, deren Metabolite und Abbauprodukte aus Blut, Blutersatzmitteln oder Lösungen zum Einbringen in den menschlichen und/oder tierischen Blutkreislauf entfernt, speziell für die wirksame Behandlung von Sepsis.

Die gestellte Aufgabe wird durch die technische Lehre des unabhängigen Anspruchs der vorliegenden Erfindung gelöst. Weitere vorteilhafte Ausgestaltungen der Erfindung ergeben sich aus den abhängigen Ansprüchen, der Beschreibung sowie den Beispielen.

Es hat sich überraschend herausgestellt, dass die extrakorporale Entfernung von Endotoxinen aus Blut durch Adsorption an eine endotoxinaffine Substanz zur Behandlung der Sepsis und die extrakorporale Organersatz- bzw. - unterstützungstherapie gleichzeitig unter Verwendung desselben Geräts und Adsorbersäule erfolgreich erfolgen kann, so dass eine kombinierte Vorrichtung zwei Funktionen gleichzeitig ausführen kann. Dies führt zu einer erheblichen Verbesserung der Therapie in verschiedener Hinsicht. Dem Patienten werden mit der gleichen Vorrichtung und mit einer einzigen Anwendung zum einen die lebensbedrohlichen Endotoxine aus dem Blut entnommen und zum anderen wird das sepsisinduziert erkrankte Organ solange unterstützt bis es aufgrund der sinkenden Endotoxinkonzentration seine Aufgabe wieder erfüllen kann. Damit ergibt sich für diese Erfindung die optimale Kopplung einer aktiven heilenden Wirkung und einer die Lebensfunktion unterstützenden Komponente. Zudem liegt mit diesem System die Belastung für den Patienten weit unter der der zur Zeit üblichen Vorgehensweise, was ebenfalls die Heilungschancen des Todkranken erhöht. Ein weiterer wichtiger Vorteil bei der Anwendung eines solchen Doppelsystems liegt in der Zeitersparnis. Wie oben bereits erwähnt, kann der akut lebensbedrohliche Zustand innerhalb von Minuten eintreten, so dass kaum Zeit für eine weitere therapeutische Maßnahme bleibt. Solche Situationen werden bei der Anwendung der doppelfunktionellen Vorrichtung von vornherein vermieden. Folglich lässt sich bei rechtzeitiger Behandlung mit der hier beschriebenen Erfindung ein akuter Sepsisschock vermeiden und damit das Leben des Patienten retten.

So erfolgt in einer bevorzugten Ausführungsform der vorliegenden Erfindung bei sepsisinduzierter eingeschränkter Lungenfunktion durch die extrakorporale Membranoxygenierung (ECMO), bei der ein Membranoxygenator den Austausch von Sauerstoff und Kohlendioxid im Blut durchführt, die Beschichtung dieser Oxygenatormembran mit Endotoxin-bindenden Substanzen für die Entfernung der Endotoxine aus dem Blut und damit für die Eliminierung der Sepsis-auslösenden Toxine. Selbstverständlich kann die beschichtete Oxygenatormembran auch nur als Endotoxinadsorber eingesetzt werden. Diese bevorzugte Ausführungsform kann als ein Oxygenator-Endotoxinadsorber bezeichnet werden.

Auf diese Weise erhält der Einsatz einer extrakorporalen Organunterstützungsapparatur eine Doppelfunktion. Zum einen werden die notwendigen Maßnahmen bei Organdysfunktion durchgeführt und gleichzeitig werden ohne weiteren Aufwand während der extrakorporalen Sauerstoffanreicherung des Blutes mit der gleichen Anlage und dem gleichen Membranmodul auch die Endotoxine aus dem Blut gefiltert und damit eine therapeutisch wichtige Maßnahme zur Heilung des Patienten erreicht.

Ebenso lassen sich bei einer anderen bevorzugten Ausführugnsform der vorliegenden Erfindung auch Hämofiltration als Nierenersatztherapie mit der extrakorporalen Oxygenierung und der Entfernung von Endotoxinen zur Dreifachfunktion kombinieren, wobei die Membran der Oxygenierung oder/und die Filtrationsmembran der Hämofiltration mit endotoxinbindenden Substanzen beschichtet ist. Die Vorrichtung mit Dreifachfunktion ist also neben der Unterstützung der Nierenfunktion und der Lungenfunktion ebenfalls in der Lage, Endotoxine aus dem Blut zu eliminieren.

Eine erfindungsgemäße Vorrichtung erfüllt also eine Doppelfunktion. Eine ihrer beiden Funktionen besteht in der Reinigung von Blut, Blutersatzmitteln oder Lösungen zum Einbringen in den menschlichen und/oder tierischen Blutkreislauf. Ihre zweite Funktion besteht in dem Gasaustausch, also der Anreicherung mit Sauerstoff und der Entfernung von Kohlendioxid, in Blut, Blutersatzmitteln oder Lösungen zum Einbringen in den menschlichen und/oder tierischen Blutkreislauf. Beide Funktionen erfüllt die Vorrichtung gleichzeitig.

Diese erfindungsgemäße Vorrichtung mit Doppelfunktion umfasst eine Säule I mit einem Einlass und gegebenenfalls einem Auslass für Gase oder Gasgemische, einem Einlass und einem Auslass für Blut, Blutersatzmittel oder Lösungen zum Einbringen in den menschlichen und/oder tierischen Blutkreislauf, mindestens einer gasdurchlässigen Membran, und einem Träger, der mit Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metabolite und Abbauprodukte beschichtet ist.

Dabei kann die Säule I zusätzlich zu dem mindestens einen Einlass und zu dem optional mindestens einen Auslass für Gase oder Gasgemische auch mehrere Einlässe aufweisen und/oder mehrere Auslässe. Weiter kann die Säule einen oder mehrere Einlässe und/oder einen oder mehrere Auslässe für Blut, Blutersatzmittel oder Lösungen zum Einbringen in den menschlichen und/oder tierischen Blutkreislauf enthalten.

Unter dem Begriff "Blut" werden Blut, Vollblut, Blutplasma und Blutserum verstanden. Unter dem Begriff "Blutersatzmittel" werden Blutersatzmittel verstanden, die z.B. wenigstens teilweise den Sauerstofftransport aktiv übernehmen können, und Volumenexpander die das noch vorhandene Restblut verdünnen und soweit ergänzen, dass der Blutkreislauf wieder funktionieren kann, aber selbst keine physiologische Funktion des Blutes übernehmen können.

Unter dem Begriff "Lösungen zum Einbringen in den menschlichen und/oder tierischen Blutkreislauf' werden intravenös, intraarteriell oder intrakardial zu verabreichende Lösungen, pharmazeutische Zubereitungen und pharmazeutische Konzentrate verstanden wie beispielsweise physiologische Kochsalzlösung, künstliche Nährmedien zur künstlichen Ernährung, Kontrastmittel insbesondere für bildgebende Verfahren wie z.B. Röntgenkontrastmittel als auch Injektionslösungen, welche pharmazeutische Wirkstoffe wie z.B. antiproliferative oder antiinflammatorische oder antiangiogene oder antivirale oder antibakterielle oder antiparasitäre Wirkstoffe.

Die erfindungsgemäße Vorrichtung mit Doppelfunktion besteht aus einer Säule I, die durch eine gasdurchlässige Membran in eine erste Kammer und eine zweite Kammer unterteilt wird. Dabei wird die erste Kammer durch den Innenraum der Säule gebildet. Die gasdurchlässige Membran kann aus einem oder mehreren Bündeln Hohlfasern bestehen. In dem Fall, dass die gasdurchlässige Membran in Form eines oder mehrerer Bündel Hohlfasern vorliegt, wird die zweite Kammer durch den Innenraum des einen oder mehrerer Bündel Hohlfasern gebildet, die in der Säule angeordnet sind. Das oder die Bündel von Hohlfasern sind so angeordnet, dass eines ihrer Enden in mindestens einen der Einlässe münden und das andere Ende in mindestens einen der Auslässe. Auf diese Weise kann die zweite Kammer von Blut bzw Blutersatzmitteln bzw. Lösungen zum Einbringen in den menschlichen und/oder tierischen Blutkreislauf oder Gas bzw. einem Gasgemisch durchflossen werden. Der Innenraum der Säule ist ebenfalls an mindestens einen Einlass und/oder mindestens einen Auslass angeschlossen, so dass auch die erste Kammer von Blut bzw. Blutersatzmitteln bzw. Lösungen zum Einbringen in den menschlichen und/oder tierischen Blutkreislauf oder Gas bzw. einem Gasgemisch durchflossen wird. Die Säule hat im Wesentlichen eine zylindrische Form; jedoch sind auch andere zweckmäßige Formen möglich.

Dabei sind zwei Ausführungsformen möglich. In der einen Ausführungsform wird die erste Kammer von Blut, Blutersatzmitteln oder Lösungen zum Einbringen in den menschlichen und/oder tierischen Blutkreislauf durchflossen und die zweite Kammer von Gas bzw. einem Gasgemisch. In der anderen Ausführungsform wird die zweite Kammer von Blut, Blutersatzmitteln oder Lösungen zum Einbringen in den menschlichen und/oder tierischen Blutkreislauf durchflossen und die erste Kammer von Gas bzw. einem Gasgemisch.

Der Träger, der mit Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metabolite und Abbauprodukte beschichtet ist, kann die Form von Partikeln oder die Form von Hohlfasern besitzen. Der Träger, der mit Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metabolite und Abbauprodukte beschichtet ist, wird im folgenden kurz als Träger bezeichnet. Liegt der Träger in Form von Partikeln vor, befinden sich die Träger-Partikel in den beiden oben aufgeführten Ausführungsformen jeweils in der Kammer, die von Blut, Blutersatzmitteln oder Lösungen zum Einbringen in den menschlichen und/oder tierischen Blutkreislauf durchflossen wird. Die von Gas durchflossene Kammer enthält dagegen keine Träger-Partikel. Liegt der Träger in Form von Hohlfasern vor, sind der Träger und die gasdurchlässige Membran zu einer Einheit zusammengefasst bzw. bilden eine Einheit.

Daneben kann die erfindungsgemäße Vorrichtung über eine dritte Funktion verfügen. Die dritte Funktion besteht dabei in der Unterstützung der Nierenfunktion durch eine Hämofiltration. Diese erfindungsgemäße Vorrichtung mit Dreifachfunktion führt also die Unterstützung der Nierenfunktion, die Unterstützung der Lungenfunktion und die Entfernung von Toxinen biologischer und chemisch-synthetischer Herkunft, deren Metabolite und Abbauprodukte aus Blut, Blutersatzmitteln oder Lösungen zum Einbringen in den menschlichen und/oder tierischen Blutkreislauf durch.

Die erfindungsgemäße Vorrichtung mit Dreifachfunktion umfasst einerseits die oben geschilderte Säule, die im folgenden als Säule I bezeichnet wird, sowie andererseits eine weitere Säule, die im folgenden als Säule **II** bezeichnet wird. Die Säule **II** umfasst ihrerseits einen oder mehrere Auslässe für Filtrat, mindestens eine semipermeable Membran, und einen Träger, der mit Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metabolite und Abbauprodukte beschichtet ist. Weiter kann die Säule **II** einen oder mehrere Einlässe und/oder einen oder mehrere Auslässe für Blut, Blutersatzmittel oder Lösungen zum Einbringen in den menschlichen und/oder tierischen Blutkreislauf enthalten. In der Vorrichtung mit Dreifachfunktion liegen die beiden Säulen, Säule **I** und Säule **II**, hintereinandergeschaltet vor, d. h. das Blut, die Blutersatzmittel oder die Lösungen zum Einbringen in den menschlichen und/oder tierischen Blutkreislauf durchlaufen zunächst die eine Säule und dann die andere Säule. Dabei kann wahlweise Säule **I** als erste durchlaufen und dann Säule **II** durchlaufen werden oder die beiden Säulen werden in der umgekehrten Reihenfolge durchlaufen. Hierbei durchläuft das Blut, die Blutersatzmittel oder die Lösungen zum Einbringen in den menschlichen und/oder tierischen Blutkreislauf bevorzugt Säule **II** (Hämofiltration und gegebenenfalls Entfernung von Toxinen) vor Säule I (Sauerstoff/Kohlendioxidaustausch und Entfernung von Toxinen).

Die Vorrichtung mit Dreifachfunktion kann also wahlweise nur Säule **I** zur Reinigung von Blut, Blutersatzmitteln oder Lösungen zum Einbringen in den menschlichen und/oder tierischen Blutkreislauf bzw. der Entfernung von Toxinen biologischer und chemisch-synthetischer Herkunft, deren Metabolite und Abbauprodukte aus Blut, Blutersatzmitteln oder Lösungen zum Einbringen in den menschlichen und/oder tierischen Blutkreislauf einsetzen. Oder die Vorrichtung mit Dreifachfunktion kann zusätzlich zu Säule I auch Säule II für diese Funktion einsetzen. Dadurch wird die Bindungskapazität der Vorrichtung mit Dreifachfunktion für Toxine biologischer und chemisch-synthetischer Herkunft, deren Metabolite und Abbauprodukte verdoppelt und der Reinigungseffekt deutlich heraufgesetzt.

Die Säule **II** ist durch eine semipermeable Membran in eine erste Kammer und eine zweite Kammer unterteilt. Dabei wird die erste Kammer durch den Innenraum der Säule gebildet. Die semipermeable Membran kann aus einem oder mehreren Bündeln Hohlfasern bestehen. In dem Fall, dass die semipermeable Membran in Form eines oder mehrerer Bündel Hohlfasern vorliegt, wird die zweite Kammer durch den Innenraum des einen oder mehrerer Bündel Hohlfasern gebildet, die in der Säule angeordnet sind. Das oder die Bündel von Hohlfasern können so angeordnet sein, dass eines ihrer Enden in mindestens einen der Einlässe münden und das andere Ende in mindestens einen der Auslässe. In dieser Anordnung fließt das Blut, das Blutersatzmittel oder die Lösung zum Einbringen in den menschlichen und/oder tierischen Blutkreislauf durch die zweite Kammer. Sind das oder die Bündel von Hohlfasern so angeordnet, dass beide ihrer Enden in mindestens einen der Auslässe münden, dient die zweite Kammer zum Auffangen und Ableiten des Filtrats. Der Innenraum der Säule **II** kann ebenfalls an mindestens einen Einlass und/oder mindestens einen Auslass angeschlossen sein, so dass auch die erste Kammer von Blut, Blutersatzmitteln oder Lösungen zum Einbringen in den menschlichen und/oder tierischen Blutkreislauf durchflossen wird. Verfügt der Innenraum der Säule über mindestens einen Auslass, dient die erste Kammer zum Auffangen und Ableiten des Filtrats. Die Säule II hat im Wesentlichen eine zylindrische Form; jedoch sind auch andere zweckmäßige Formen möglich.

Dabei sind zwei Ausführungsformen möglich. In der einen Ausführungsform wird die erste Kammer von Blut, Blutersatzmitteln oder Lösungen zum Einbringen in den menschlichen und/oder tierischen Blutkreislauf durchflossen und die zweite Kammer dient zum Auffangen und Ableiten des Filtrats. In der anderen Ausführungsform wird die zweite Kammer von Blut, Blutersatzmitteln oder Lösungen zum Einbringen in den menschlichen und/oder tierischen Blutkreislauf durchflossen und die erste Kammer dient zum Auffangen und Ableiten des Filtrats.

Der Träger, der mit Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metabolite und Abbauprodukte beschichtet ist, kann die Form von Partikeln oder die Form von Hohlfasern besitzen. Der Träger, der mit Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metabolite und Abbauprodukte beschichtet ist, wird im folgenden kurz als Träger bezeichnet. Liegt der Träger in Form von Partikeln vor, befinden sich die Träger-Partikel in den beiden oben aufgeführten Ausführungsformen jeweils in der Kammer, die von Blut, Blutersatzmitteln oder Lösungen zum Einbringen in den menschlichen und/oder tierischen Blutkreislauf durchflossen wird. Die Kammer, die zum Auffangen und Ableiten des Filtrats dient, enthält dagegen keine Träger-Partikel. Liegt der Träger in Form von Hohlfasern vor, sind der Träger und die semipermeable Membran zu einer Einheit zusammengefasst bzw. bilden eine Einheit.

Beide Vorrichtungen umfassen weiter diverse Schlauchverbindungen, eine Filtereinheit, eine Pumpe und nicht notwendiger- aber vorteilhafterweise eine Temperiereinheit. Die Temperiereinheit sorgt dafür, dass die Temperatur des Bluts, der Blutersatzmittel oder der Lösungen zum Einbringen in den menschlichen und/oder tierischen Blutkreislauf auf Körpertemperatur gehalten wird oder .je nach den Erfordernissen erhöht oder verringert wird. Die Filtereinheit hat die Aufgabe Teilchen, welche aus der Vorrichtung in das Blut, die Blutersatzmittel oder die Lösungen zum Einbringen in den menschlichen und/oder tierischen Blutkreislauf übergegangen sein könnten, oder überschüssiges Gas aus dem Blut, den Blutersatzmitteln oder den Lösungen zum Einbringen in den menschlichen und/oder tierischen Blutkreislauf abzutrennen, bevor das Blut, die Blutersatzmittel oder die Lösungen zum Einbringen in den menschlichen und/oder tierischen Blutkreislauf zum Patienten zurückgeleitet wird. Die Pumpe dient der kontinuierlichen Förderung des Blutes, der Blutersatzmittel oder der Lösungen zum Einbringen in den menschlichen und/oder tierischen Blutkreislauf vom Patienten zur Vorrichtung und wieder zum Patienten zurück. Die Vorrichtung mit Dreifachfunktion umfasst zwei weitere Pumpen, welche für die Abführung des Filtrats sowie die Zuführung der Substitutionsflüssigkeit zuständig sind.

Die erfindungsgemäßen Vorrichtungen arbeiten extrakorporal, d.h. Blut wird einem Patienten kontinuierlich entnommen, die Reinigung des Bluts und/oder der Gasaustausch und/oder der Flüssigkeitsaustausch finden außerhalb des Patienten in einer der Vorrichtungen statt und das behandelte Blut wird kontinuierlich dem Patienten wieder zugeführt.

Die semipermeable Membran ist im Wesentlichen durchlässig für Elektrolyte, Harnstoff, Kreatinin, Phosphat, Aminosäuren, Medikamente und Wasser.

Die gasdurchlässige Membran der Vorrichtung mit Doppelfunktion ist im Wesentlichen durchlässig für Sauerstoff und Kohlendioxid, aber auch für andere Gase. Die gasdurchlässige Membran ist jedoch nicht durchlässig für Flüssigkeiten. Die gasdurchlässige Membran und die semipermeable Membran werden im folgenden kurz als Membran bezeichnet. Die Membran kann als flächige Folie oder Stapel von Folien oder als ein oder mehrere Bündel Hohlfasern vorliegen. Die Membran bzw. die Hohlfasern bestehen aus einem Material oder Polymer aus der folgenden Gruppe:

Polyolefine, Polyethylen (HDPE, LDPE, LLPE), fluoriertes Polyethylen, Copolymere des Ethylens mit Buten-(1), Penten-(1), Hexen-(1), Copolymere des Ethylens und Propylens, EPR-Kautschuk oder EPT-Kautschuk (dritte Komponente mit Dienstruktur u. a. Dicyclopentadien, Ethylidennorbonen, Methylendomethylenhexahydronaphthalin, cis-cis-Cyclooctadien-1,5-Hexadien-1,4), Hexyo-(1-Hexen-methylhexadien), Ethylen-Vinylacetat-Copolymer, Ethylen-Methacrylsäure-Copolymer, Ethylen-N-Vinylcarbazol, Methacrylamid-N,N'-Methylen-bis(meth)acrylamid-Allylglycidylether, Glycidyl(meth)acrylat), Polymethacrylat, Polyhydroxymethacrylat, Styrol-Glycidylmethacrylat-Copolymere, Polymethylpenten, Poly(methyl methacrylatmethacryloylamidoglutaminsäure), Poly(glycidyl methacrylat-co-ethylen dimethacrylat), Styrol-Polyvinylpyrrolidon-Glycidylmethacrylat-Copolymer, Styrol-Polyvinylpyrrolidon-Blends mit Crospovidone, Ethylen-Trifluorethylen, Polypropylen, Polybuten (1), Poly-4- (Methylpenten (1)), Polymethylpentan, Polyisobutylen-Copolymer, Isobutylen-Styrol-Copolymer, Butylkautschuk, Polystyrol und modifiziertes Styrol, chlormethyliertes Styrol, sulfoniertes Styrol, Poly-(4-Aminostyrol), Styrol-Acrylnitril-Copolymer, Styrol-Acrylnitril-Butadien-Copolymer, Acrylnitril-Styrol-Acrylester-Copolymer, Styrol-Butadien-Copolymer, Styrol-Divinylbenzol-Copolymer, Styrol-Maleinsäureanhydrid-Copolymer, Polydiene in der cis-trans, in der 1-2 und in der 3-4 Konfiguration, Butadien, Isopren, gereinigter Naturkautschuk, Chloroporem, Styrol-Butadien-Copolymer (SBR), Triblockpolymere (SBS), NBR Acrylnitril-Butadien-Copolymer, Poly-(2,3-dimethylbutadien), ein Triblock-Copolymer aus Poiybutadien terminiert mit cycloaliphatischen sekundären Aminen, oder -benzal-L-glutamat oder Polypeptiden, oder N-Carbobenzoxylysin, Poly-(alkenamere)-Polypentenamer, Poly-(1-hexebmethyl-hexadien), Poly-Phenylene, Poly-(p-xylylen), Polyvinylacetat, Vinylacetat-Vinylstearat-Copolymer, Vinylacetat-Vinylpivalat-Copolymer, Vinylacetat-Vinylchlorid-Copolymer, Polyvinylalkohol, Polyvinylformal, Polyvinylbutyral, Polyvinylether, Poly-(N-vinylacarbazol), Poly-N-vinylpyrrolidon, Poly-(4-vinylpyridin), Poly-(2-vinylpyridiniumoxid), Poly-(2-methyl-5-vinylpyridin), Butadien-(2-methyl-5-vinylpyridin)-Copolymer, Polytetrafluorethylen, Tetrafluorethylen-Hexafluorpropylen-Copolymer, Tetrafluorethylen-Perfluorpropylvinylether-Copolymer, Tetrafluorethylen-Ethylen-Copolymer, Tetrafluorethylen-Trifluornitrososmethan-Copolymer, Tetrafluorethylen-Perfluormethylvinylether-Copolymer, Tetrafluorethylen-(Perfluor-4-Cyanobutylvinylether)-Copolymer, Poly-(trifluorchlormethylen, Trifluorchlorethylen-Ethylen-Copolymer, Polyvinylidenfluorid, Hexafluorisobutylen- vinylidenfluorid-Copolymer, Polyvinylfluorid, Polyvinylchlorid, chloriertes PE, FVAC oder Polyacrylaten, Weich-PVC, nachchloriertes PVC, Polyvinylchlorid-Vinylacetat-Copolymer, Vinylchloridpropylen-Copolymer, Polyvinylidenchlorid-Vinylchlorid-Vinylchlorid- Vinylidenchlorid-Copolymer, Vinylidenchlorid-acrylnitril-Copolymer, Polyacrylsäure, Acrylsäure-Itakonsäure-Copolymer, Acrylsäure-Methacrylsäure-Copolymer, Acrylsäureester-acrylnitril-Copolymer, Acrylsäureester-2-chlorethylenvinylether-Copolymer, Poly-(1,1-dihydroperfluor-butylacrylat), Poly-(3-perfluormethoxy-1,1-dihydroperfluorpropylacrylat), Polysulfon, Polyacrolein, Polyacrylamid, Acrylsäure-Acrylamid-Copolymer, Acrylamid-maleinsäure-Coplymer, Acrylamid-hydroxymethylmethacrylat-Copolymer, Acrylamid-methylmethacrylat-Copolymer, Acrylamid-methylacrylat-Copolymer, Acrylamid-maleinsäureanhydrid-Copolymer, Acrylamid-methacrylsäureanhydrid-Copolymer, Acrylamid-anilino-Acrylamid-Copolymer, Acrylamid-(N-acrylol-4-carboxymethyl-2,2-dimethylthiazoline)-Copolymer, Polymethacrylamid, Methacrylsäure-methacrylnitril-Copolymer, Methacrylsäure-3-fluorstyrol-Copolymer, Methacrylsäure-4-fluorstyrol-Copolymer, Methacrylsäure-3-fluoranilid-Copolymer, nitrierte Copolymere von Methacrylsäure mit Methacrylsäure-3-fluoroanilid oder Fluorostyrol oder Copolymere von Methacrylsäure mit 3,4-Isothiocyanatostyrol, oder N-Vinylpyrolidon mit Maleinsäureanhydrid, oder Polyvinylalkohol und Polyallylalkohol, Polyacrylnitril, Acrylnitril-2-Vinylpyridin-Copolymer, Acryl-nitril-methallylsulfonat-Copolymer, Acrylnitril-N-Vinylpyrrolidon-Copolymer, hydroxylgruppenhaltiges PAN, Acrylnitril-vinylacetat-Copolymer, Acrylnitril-acrylester-Copolymer, Polyallylverbindungen, Polydiallylphthalate, Polytrisallylcyanurat, Poly-α-cyanoacrylat, Polydimethylaminoethylmethacrylat und Coplymere mit Acrylnitril, Methylmethacrylatlaurylmethacrylat-Copolymer, P-Acetaminophenylethoxymethacrylat-methylmethacrylat-Copolymer, Glycoldimethylmethacrylat-methacrylat-Copolymer, Poly-2-hydroxyethylmethacrylat, 2-Hydroxymethylmethacrylat-methylmethacrylat-Copolymer, Glycoldimethacrylat-methacrylat- Copolymer, Poly-2-hydroxymethylmethacrylat, 2-Hydroxymethylmethacrylat-methylmethacrylat-Copolymer, Glycolmethacrylat-glycoldimethylmethacrylat-Copolymer, Hema-Styrol-Block und Pfropf-Copolymere, Poly-N,N-P,P-oxydiphenylenmellitimid, Polydiethylenglycolbisallylcarbonat, aliphatische Polyether, Polyoxymethylene, Polyoxyethylene, Polyfluoral, Polychloral, Polyethylenoxyd, Polytetrahydrofuran, Polypropylenoxyd, Ethylenoxydpropylenoxyd-Copolymer, Propylenoxyd-allylglycidylether-Copolymer, Polyepichlorhydrin, Ethylenoxid-epichlorhydrin-Copolymer, Poly-1,2-dichlormethyl-ethylenoxid, Poly-2,2-bis-chlormethyl-oxacyclobutan, Epoxid-Harze, Bis-phenol-A-diglycidylether, epoxidiertes Phenol-Formaldehyd, Kresol-Formaldehyd, Harze, Vernetzung mit Carbonsäureanhydriden, Aminen wie Diethylentnamin, Isophorondiamid, 4,4-Diaminodiphenyl-methan, aromatische Polyether, Polyphenylenoxide, Polyphenol, Phenoxyharze, aliphatische Polyester, Polylactid, Polyglycolid, Poly-b-Propionsäure, Poly-b-D-hydroxybutyrat, Polypivolacton, Poly-e-caprolacton, Polyethylenglycoladipat, Polyethylenglycolsebazat, ungesättigte Polyester aus Maleinsäureanhydrid, Phthalsäureanhydrid, Isophthalsäure, Terephthalsäure oder HET-Säure mit, Ethylenglycol, 1,2-Propylenglycol, Neopentylglycol, oxethylierte Bisphenole oder Cyclododecandiol Vernetzung ungesättigter Polyester-Harze oder Vinylesterharze durch Copolymerisation von ungesättigten Polyestern mit Styrol, Methacrylat, Vinylmonomere, Vinylacetat, Methylmethacrylat, Polycarbonat aus Bisphenol A sowie dessen Derivate und Polyether, Polyester, segmentierte Polycarbonate aus Bisphenol A sowie dessen Derivate und aliphatische Polyether, sowie aliphatische Polyester (siehe oben), Polyethylenglykolterephthalat (PET) oberflächenmodifiziert, mit Acrylsäure gepfropft oder durch partielle Hydrolyse der Oberfläche von PET, Polyethylenglykolterephthalat; Polyethylenglykolterephthalatadipat, Polyethylenglykolterephthalat, segmentiert mit Polyetherblöcken und aliphatischen Polyesterblöcken und Pölytetrahydrofuranblöcken, Poly-p-Hydroxybenzoat, Hydroxybenzoesäure-hydrochinon-Copolymer, Hydroxybenzoesäure-terephthalsäure-Copolymer, Hydroxybenzoesäure-p,p-Diphenylether-Copolymer, Polyvinylpyrrolidon, Polyvinylpyrrolidonmaleinsäureanhydrid-Copolymer, Alkydharze aus Glycerin, Pentaerythrit, Sorbit mit Phthalsäure, Bernsteinsäure, Maleinsäure, Fumarsäure, Adipinsäure und Fettsäure aus Leinöl, Ricinusöl, Sojaöl, Kokosöl, aliphatische Polysulfide-(R-Sx-) = Schwefelgrad, aromatische Polysulfide, Polythio-1,4-phenylen, aromatische Polysulfidether aus Phenol und Thiophen, Polyethersulfone, Polysulfo-1,4-phenylen, Poly-p-Phenylensulfon, Polyimine, Polyethylenimine, verzweigte Polyethylenimine, Polyalkylenamine, Polyamide, Polyhexamethylenadipamid; Polyhexamethylensebacamid, Polyhexamethylendodekandiamid, Polytridekanbrassylamid, Versamide aus pflanzlichen Ölen mit Diaminen und Triaminen, Polyamid aus ω-Aminocarbonsäuren mit α-, ß-, γ-, δ- Aminocarbonsäuren oder Lactamen, Terephthalsäure-m-aminobenzamid-Copolymer, Terephthalsäurephenylendiamin-Copolymer, Polyamidhydrazide, z B. aus Isophthalsäure und m-Aminobenzhydrazid, Polypiperazinamide, z. B. aus Fumarsäure und Dimethylpiperazin, Polybenzimidazole aus Terephthalsäure und Tetraaminobenzol (substituiert), oder aus Diaminodiphenylether und Dichlordiphenylsulfon (substituiert und cyclisiert) oder aus m-Phenylenisophthalamid und Terephthalamid, Polyimide z. B. aus Pyromellitsäuredianhydrid, Methoxy-m-phenylendiamin, Pyrone z. B. aus Pyromellitsäuremedianhydrid und Diaminobenzidin, aromatische Polyamide, Poly-m-phenylenisophtalamid, Poly-p-benzamid, Poly-p-phenylenterephthalamid, m-Aminobenzoesäure-p-phenylendiamin-isophthalsen-Copolymer, Poly-4,4-diphenylsulfonterephthalamid, aus Terephthalsäure und Hexamethylentetramin, Terephthalsäure und Gemischen aus 2,4,4-Trimethylhexamethylendiamin und 2,4,4-Trimethylhexamethylendiamin, aus Terephthalsäure, Diaminomethylennorbonen und ε-Caprolactam, aus Isophthalsäure und Laurinlactam, aus Isophthalsäure und Di-4-(cyclohexylamino-3-methyl)-methan, aus 1,12-Decandisäure und 4,4-Diaminodicyclohexylmethan, aromatische Polyamide mit Heterocyclen aus Dicarbonsäurechlorid, Terephthalsäure und Isophthalsäure, diaminhaltige Heterocyclen, mit Oxdiazol, Triazol Bithiazol und Bezimidazol Strukturen, 3-(p-Aminophenyl)-7-amino-2,4-(1 H,3H)-chinazolindion und Isophthalsäure, Polyaminosäuren, Polymethyl-L-glutamat, Poly-L-glutaminsäure u. a. Copolypeptide, z B. Glutaminsäure und Leucin, Glutaminsäure und Phenylalanin, Glutaminsäure und Valin, Glutaminsäure und Alanin, Lysin und Leucin, p-Nitro-D,L-phenylalanin und Leucin u. a., Polyharnstoffe aus Diisocyanaten mit Diaminen und Harnstoffen, Polyurethane aus aliphatischen und aromatischen Diisocyanaten und bifunktionellen und trifunktionellen hydroxylhaltigen aliphatischen Polyestern (siehe oben) und aliphatischen Polyethern (siehe oben) und gegebenenfalls Modifizierungen mit bifunktionellen aminogruppenhaltigen, hydroxylgruppenhaltigen und carboxylhaltigen Substanzen, z. B. Hexamethylendiisocyanat, Diphenylmethandiisocyanat, Tolylendiisocyanat, 2,4- und 2,6-Tolidindiisocyanat, Xylylendiisocyanat, Glycerin, Ethylenglycol, Diethylenglycol, Pentaerithrit, 3-Dimethylamin-12-propandiol und Kohlenhydrate, aliphatische und aromatische Dicarbonsäuren und deren Derivate, o,p,m-Phenylendiamin, Benzidin, Methylen-bis-o-chloroanilin, p,p-Diaminodiphenylmethan, 1,2-Diaminopropan, Ethylendiamin, Aminoharze aus Harnstoff und cyclischen Harnstoffen, Melamin, Thioharnstoff, Guanidin, Urethan, Cyanamid, Säureamide und Formaldehyd, sowie höhere Aldehyde und Ketone, Silikone, Polydialkylsiloxan, Diarylsiloxan und Alkyl-arylsiloxan wie Dimethyl-, Diethyl-, Dipropyl-, Diphenyl-, Phenylmethyl-Siloxan, Silikone mit funktionellen Gruppen, z B. Allylgruppen, γ-substituierte Fluorsilikone mit Aminogruppen und Vinylgruppen, z. B. aus Aminopropyltriethoxysiloxan, 2-Carboxylpropylmethylsiloxan, Blockpolymer mit Dimethylsiloxaneinheiten und Polystyrol- oder Polycarbonatblöcken, Dreiblock Copolymere aus Styrol, Butylacrylat mit α,ω-Dihydroxy-Polydimethylsiloxan, 3,3,3-Trifluorpropylmethylsiloxan, Avocane (90 Silikon und Polycarbonat), hydrophobe Polymere unter Zusatz von hydrophilen Polymeren, z.B. Polysulfon-Polyvinylpyrrolidon; Cellulose und Cellulosederivate, z. B. Celluloseacetat, Perfluorbutyrylethylcellulose, Perfluoracetylcellulose, polyaromatische Polyamidpolymere, Cellulosenitrat, Carboxymethylcellulose, regenerierte Cellulose, regenerierte Cellulose aus Viskose, und ähnliche Cellulosederivate, Agarose, Polysaccharide wie Carragenane, Dextrane, Mannane, Fructosane, Chitin, Chitosan-(ethylene glycol diglycidyl ether) (Chitosan-EGDE), Chitosan, Pectine, Glycosaminoglycane, Stärke, Glycogen, Alginsäure, sowie alle Deoxypolysaccharide und deren Derivate, Murein, Proteine, z B. Albumin, Gelatine, Kollagen I-XII, Keratin, Fibrin und Fibrinogen, Casein, Plasmaproteine, Milchproteine, Crospovidon, Strukturproteine aus tierischen und pflanzlichen Geweben, Sojaproteine, Proteine aus der Nahrungsmittelindustrie.

Zusätzliche Materialien oder Polymere erhält man, indem die oben aufgeführten Polymere, die aus den verschiedenen Monomerbausteinen synthetisiert werden, mit weiteren Monomeren copolymerisiert werden, wie sie in "Functional Monomers", Ed. R H. Yocum und E B. Nyquist, Vol. I und II: Marcel Dekker, New York 1974, aufgeführt sind. Ferner können die oben aufgeführten Polymere durch Pfropfung und durch Herstellung von weiteren Block-Copolymeren und Pfropf-Copolymeren partiell oder voll modifiziert werden. Außerdem können Polymermischungen, beschichtete Polymere und Polymere in Form verschiedener Verbundwerkstoffe hergestellt werden. Weiterhin können Polymerderivate mit bi- und polyfunktionellen Vernetzungsreagenzien hergestellt werden, wie sie für die Herstellung von reaktionsfähigen Polymeren von den Methoden der Peptid-, Protein-, Polysaccharid- und Polymerchemie bekannt sind.

Dabei werden hydrophobe Polymere bevorzugt. Besonders bevorzugt sind Membranen bzw. Hohlfasern bestehend aus folgenden Materialien oder Polymeren:

Silica, Silikone, Polyolefine, Polytetrafluorethylen, Polyesterurethane, Polyetherurethane, Polyurethane, Polyethylenterephthalate, Polymethylpentan, Polymethylpenten, Polysaccharide, Polypeptide, Polyethylene, Polyester, Polystyrole, Polyolefine, Polysulfonate, Polypropylen, Polyethersulfone, Polypyrrole, Polyvinylpyrrolidone, Polysulfone, Polymilchsäure, Polyglycolsäure, Polyorthoester, polyaromatische Polyamide, Aluminiumoxid, Gläser, Sepharose, Kohlenhydrate, Copolymere von Acrylaten oder Methacrylaten sowie Polyamiden, Polyacrylsäureester, Polymethacrylsäureester, Polyacrylsäureamide, Polymethacrylsäureamide, Polymethacrylat, Polyetherimid, Polyacrylnitril, Copolymere aus Ethylenglycoldiacrylat oder Ethylenglycoldimethacrylat und Glycidylacrylat oder Glycidylmethacrylat und/oder Allylglycidether, regenerierte Cellulose, Celluloseacetat, hydrophobe Polymere unter Zusatz von hydrophilen Polymeren, z.B. Polysulfon-Polyvinylpyrrolidon, Derivate und Copolymere der genannten Polymere.

Die Länge der Hohlfasern liegt zwischen 30 - 150 mm, vorzugsweise zwischen 50 und 100 mm. Der Außendurchmesser einer derartigen Hohlfaser beträgt etwa 0,1 - 1,5 mm, der Innendurchmesser beträgt etwa 0,1 - 1 mm während die Wandstärke der Membran bzw. der Hohlfaser selbst 5 - 200 µm, vorzugsweise 15 - 50 µm beträgt.

Die Wände der Hohlfasern können Poren enthalten. Die Porosität auf der inneren und äußeren Oberfläche der Hohlfasern der gasdurchlässigen Membran liegt im Bereich von 10 bis 90 %. Die Poren besitzen einen Durchmesser im Bereich von 0 - 5 µm und bevorzugt einen Durchmesser von 0 - 1,5 µm. Allgemein sollte die Porengrösse so gering wie möglich gehalten werden, da bei längerer Anwendung der Vorrichtung mit Doppelfunktion über die Poren unerwünscht Plasma in die von Gas durchflossene Kammer eindringen kann und damit dem Patienten entzogen wird als auch die Leistung der Vorrichtung mit Doppelfunktion erniedrigt. Die Poren werden in den Faserwänden vorzugsweise geformt durch Ausdehnen oder durch Fest-Flüssigphasen-Trennung.

Die Porosität auf der inneren und äußeren Oberfläche der Hohlfasern der semipermeablen Membran liegt im Bereich von 10 bis 90 %. Die Poren besitzen vorzugsweise einen Durchmesser im Bereich von 0,01 bis 1,5 µm.

Die Hohlfasern der Membran besitzen eine innere und eine äußere Oberfläche. Die innere Oberfläche stellt dabei die Oberfläche des Lumens der Hohlfasern dar und die äußere Oberfläche besteht in der Oberfläche der Außenseite der Hohlfasern. Die gesamte Oberfläche der Hohlfasern liegt zwischen 0,1 und 6 m².

Der Träger, der mit Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metabolite und Abbauprodukte beschichtet ist, kann die Form von Partikeln oder die Form von Hohlfasern besitzen. Liegt der Träger in Form von Hohlfasern vor, sind der Träger und die gasdurchlässige Membran zu einer Einheit zusammengefasst bzw. bilden eine Einheit oder bilden gemeinsam ein untrennbares Bauteil. Der Träger in Form von Hohlfasern umfasst dabei alle vorgenannten Eigenschaften der gasdurchlässigen Membran. Der Träger in Form von Hohlfasern erfüllt in diesem Fall zwei Funktionen. Er sorgt einerseits für einen Gasaustausch, vorzugsweise einen Austausch von Sauerstoff und Kohlendioxid, zwischen dem Strom des Bluts, der Blutersatzmittel oder Lösungen zum Einbringen in den menschlichen und/oder tierischen Blutkreislauf auf der einen Seite der Hohlfaser und dem Gasstrom auf der anderen Seite der Hohlfaser. Gleichzeitig ist er mit Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metabolite und Abbauprodukte beschichtet. Dadurch führt der Träger eine zweite Funktion aus, nämlich das gleichzeitige Binden und damit Entfernen von Toxinen biologischer und chemisch-synthetischer Herkunft, deren Metabolite und Abbauprodukte aus Blut, Blutersatzmitteln oder Lösungen zum Einbringen in den menschlichen und/oder tierischen Blutkreislauf.

Als alternative Ausführungsform kann der Träger in der Form von Partikeln vorliegen. Die Partikel bestehen ebenfalls aus Polymeren. Dabei werden für die Partikel unabhängig von den Hohlfasern Polymere aus der gleichen Gruppe ausgewählt, die für die Hohlfasern aufgeführt wurde. Die folgenden Polymere werden für Partikel bevorzugt:

Methacrylamid-N,N'-methylen-bis(meth)acrylamid-allylglycidylether, Glycidylmethacrylat, Polyacrylsäure, Dextran, Regeneratcellulose, Cellulose, Polysaccharide, Polymethacrylat, Polyhydroxymethacrylat, Polysulfon, Polyethersulfon,- Styrol-Glycidylmethacrylat-Copolymere, Styrol-Polyvinylpyrrolidon-Glycidylmethacrylat-Copolymere, Silikone, Styrol-Maleinsäureanhydrid-Copolymere, Crospovidone (Popcorn-Polymere), Styrol-Polyvinylpyrrolidon-Blends mit Crospovidone, Zeolithe, MCM's (Mm/x[AlmSinO2(m+n)] pH2O), Polyamide, Polyhydroxymethacrylat, Poly(methyl-methacrylat-methacryloylamidoglutaminsäure), Chitosan-(ethylene glycol diglycidyl ether) (Chitosan-EGDE), Chitosan, Poly(glycidyl methacrylat-co-ethylen dimethacrylat), Polyvinylalkohol, Polyacrylamid.

Die Partikel können in folgenden Formen vorliegen: kugelförmig, zylindrisch, unregelmäßig, ringförmig. Die Partikel weisen einen Durchmesser von 50 µm - 5 mm auf. Der Innendurchmesser der ringförmigen Partikel liegt zwischen 20 µm - 4,5 mm. Aufgrund ihrer Größe und ihrer Formen sind die Partikel in der Lage in den Säulen der Vorrichtung Packungen auszubilden, welche Kanäle enthalten, die für die Bestandteile von Blut und Vollblut, vor allem auch für die Blutzellen, durchlässig sind. Ein Verstopfen der Partikel-Packungen in den Säulen wird auf diese Weise vermieden. Die Partikel besitzen ebenfalls eine äußere Oberfläche.

Weiter kann der Träger Poren aufweisen, und zwar sowohl in seiner Form als Partikel als auch in seiner Form als Hohlfaser bzw. Hohlfaserbündel. Liegt der Träger als Hohlfaser vor, befinden sich die Poren in seinen Wänden und durchqueren die Wände im wesentlichen vollständig, so dass die Poren Kanäle zwischen der Innenseite (der Lumenseite) und der Außenseite der Hohlfasern bilden. Durch diese Kanäle diffundieren Sauerstoff- und Kohtendioxidmoleküle. Sauerstoff- und Kohlendioxidmoleküle können auch direkt durch die Wände der Hohlfasern diffundieren.

Die Porosität von Partikeln oder Hohlfasern liegt im Bereich von 10 bis 90 %. Die Poren besitzen einen Durchmesser im Bereich von 0 - 5 µm und bevorzugt einen Durchmesser von 0 - 1,5 µm. Die Poren in Partikeln oder Hohlfasern besitzen ebenfalls eine Oberfläche, welche als innere Oberfläche der Poren oder als Oberfläche der Poren bezeichnet wird.

Die Oberflächen der Träger weisen chemische funktionelle Gruppen auf, die entweder Bestandteil des Polymers sind, aus welchem der Träger besteht, oder die durch Aktivierung, Modifizierung oder durch Reaktion mit einem Vernetzer der Oberflächen der Träger hergestellt wurden.

Die Oberflächen können aktiviert oder modifiziert werden durch energiereiche Strahlung, Belichtung, Oxidation, hydrolytischen Anbau, durch photochemische Reaktionen, Plasmabehandlung, durch Halogenierung, Sulfochlorierung, Chlormethylierung, Veresterung, Veretherung, Epoxidierung, durch Umsetzung mit Radikalbildnern, Aminen, Amiden, Imiden, Isocyanaten, Aldehyden, Ketonen, Nitrile, Vinylverbindungen, Carbonsäuren und -derivate sowie Diazoverbindungen.

Als chemische funktionelle Gruppen oder Vernetzungsmoleküle auf der Oberfläche der Träger kommen die folgenden in Frage.
Phosgen, Formaldehyd, Glyoxal, Acrolein, Glutardialdehyd, Azide, aktivierte Ester, Anhydride, Säurechloride, Ester, gemischte Anhydride, Bromcyan, Difluordinitrobenzol Thioisocyanate, Epoxide, Imide, Isocyanate, Urethiongruppen, Diisocyanate, Triisocyanate, Maleinimid, Dicyclohexylcarbodiimid, N,N-Bis-(trimethylsilylschwefeldiimid), Peroxide, Vinylketongruppen, aromatische Diazoverbindungen, Vinylsulfone, Trichlortriazin, Monochlortriazin, Dichlortriazin, Bromacrylamid, Difluorchlorpyrimidin, Trifluorpyrimidin, Dichlorchinoxalin, Chloracetylaminogruppen, Chloracetylharnstoff, ß-Halogenpropionamid, a,ß-Dihalogenpropionamid, ß-quarternäres Ammoniumpropionamid, ß-Sulfatopropionamid, ß-Sulfonylpropionamid, substituierte Alkan-Dicarboxamide, substituierte Alkan-Monocarboxylate, substituierte Cycloalkan-Carboxamide, Alken-Monocarboxamide, Arylamide, Crotonamide, substituierte Acrylamide, Mono-, Di- und Trihalogen-Arylamide, substituierte Crotonamide, Alken-dicarboxamide, cyclische Halogenmaleinimide, Alkin Carboxamide, substituierte aliphatische Ketone, Amide von substituierten aliphatischen Ketonen, Amide von substituierten aliphatischen Sulfonsäuren, substituierte Methansulfonamide, substituierte Ethansulfonamide, ß-Thiosulfatoethylsulfonamide, quarternäres Ammoniummethansulfonamid, Vinylsulfonamid, ß-Chlorvinylsulfonamid, Ester von reaktiven aliphatischen Sulfonsäuren, ß-substituierte Ethylsulfonsäure, ß-Thiosulfatoethylsulfone, ß-Halogenvinylsulfone, ß-substituierte Ethylamiraderivate, ß-Sulfatoethylamin, ß-Halogenethylpyrazolon, N-(ß-Halogen ethyl)-amide, N-(β-Sulfatoethyl)-amide, ß-substituierte Ethylammonium-Verbindungen, ß-substituierte Ethylamide von Sulfonsäure N,ß-Halogenethylsulfonamide, ß,y-Dihalogenpropionylamide von Sulfonsäuren, ß-Sulfatoethylamide von Sulfonsäuren, Ethylenimin und Ethyleniminverbindungen, Allylgruppen, Propargylgruppen, Diallylphthalat, Triallylcyanurat, Benzylderivate, 2-substituierte Thiazolcarbonsäuren, Chlorsulfonylpyridin, 4-substituiertes 3,5-dicyano-2,6-dichlorpyridin, 2,6-bis-(methylsulfonyl)-pyridin-4-carbonylchlorid, Chlorpyridazin, Dichlorpyridazon, 1-Alkyl-4,5-dichlor-6-pyridazon, Chlor- und Brompyrimidin, 3-(2,4,5-trichlorpyrimidyl-(6)amino)anilin, 4,5,6-Trichlorpyrimidin-2-carbonylchlorid, Trifluoropyrimidin, 2-Chlortriazinylderivate, 2-Chlor-4-alkyl-s-(trizinyl-6-aminocarbonsäure), 2-Chlorobenzothiazolcarbonyl, 6-Amino-2-fluorbenzothiazoi, 2-Methylsulfonyl-6-aminobenzothiazole, 2,3-Dichlorchinoxalin-6-carbonylchlorid, 1,4-Dichlorphthalazin-6-carbonylchlorid, 3-Chloro-1,2,3-benzotriazin-1-N-oxid-7-carbonylchlorid, Fluor-2-nitro-4-azidobenzol, Sulfonsäureester, N-Sulfonylharnstoffe, Thiosulfato-S-Alkylester, N-Methylthylolharnstoff, N,N-dimethylol-glyoxal-monourein, Terephthaldialdehyd, Mesitylentrialdehyd, Isothiuroniumgruppen, Triacylformal, 4-Azido-1-fluoro-2-nitrobenzol, N-(4-Azido-2-nitrophenyl)-1,1-aminoundecansäure und Oligomethacrylsäure.

Bevorzugte chemische funktionelle Gruppen sind primäre Amine, welche sich mit Carbonylverbindungen zu Iminen umsetzen lassen und anschließend gegebenenfalls durch Hydrierung zu einer stabileren Aminbindung umgesetzt werden können. Darüber hinaus können an Amine Carbonsäuren über eine Amidbindung immobilisiert werden. Bevorzugt Verwendung finden zudem Aziridine, Oxirane, Maleinimide, N-Succinimidylester, N-Hydroxysuccinimide, Hydrazide, Azide, Aldehyde, Ketone, Carbonsäuren, Carbonsäureester und Epoxide.

Die chemischen funktionellen Gruppen werden genutzt, um die Substanzen zur adsorptiven Entfernung von Toxinen biologischer und chemisch-synthetischer Herkunft, deren Metabolite und Abbauprodukte auf den Oberflächen der Träger zu immobilisieren. Die Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metabolite und Abbauprodukte werden im folgenden kurz als Substanzen bezeichnet.

Folgende Kombinationen der verschiedenen Oberflächen der Träger werden gezielt mit den Substanzen beschichtet:
- Träger in Form von Hohlfasern: äußere Oberfläche oder Oberfläche des Lumens
- Träger in Form von Hohlfasern: äußere Oberfläche und Oberfläche des Lumens
- Träger in Form von Partikeln: äußere Oberfläche der Partikel
- Träger in Form von Partikeln: äußere Oberfläche und Oberfläche der Poren

Die beschichteten Oberflächen sind jeweils diejenigen der Träger, die mit dem Blut, den Blutersatzmitteln oder den Lösungen zum Einbringen in den menschlichen und/oder tierischen Blutkreislauf in direkten Kontakt kommen.

Die Immobilisierung der Substanzen erfolgt bevorzugt kovalent. Eine andersartige Anbindung, beispielsweise durch hydrophobe, elektrostatische und/oder ionische Wechselwirkungen, ist jedoch auch möglich. Die Substanzen können direkt über die chemischen funktionellen Gruppen an die Oberflächen der Träger gebunden sein.

Alternativ können an die chemischen funktionellen Gruppen auf den Oberflächen der Träger zunächst sogenannte Linker-Moleküle, auch als Spacer oder Vernetzer bezeichnet, gebunden werden. Dabei handelt es sich um langgestreckte lineare Moleküle, welche an jedem ihrer Enden über eine reaktive Funktionalität verfügen. Eines dieser Enden kann gezielt an die chemischen funktionellen Gruppen auf den Oberflächen der Träger gebunden werden. Das andere Ende steht mit seiner Funktionalität zur Verfügung, um die Substanzen auf den Oberflächen der Träger zu binden. Damit können die Substanzen über Linker an die Oberflächen der Träger gebunden sein. Als Linker können die beschriebenen linearen Verbindungen eingesetzt werden, wobei die reaktive Funktionalität ebenfalls aus der Gruppe der genannten chemischen funktionellen Gruppen ausgewählt wird. Entscheidend für die Auswahl der geeigneten reaktiven Funktionalität ist, dass sie in der Lage ist, mit der auf der Oberfläche der Träger vorhandenen chemischen funktionellen Gruppe zu reagieren und eine Bindung auszubilden. Alternativ kann als Linker eines der genannten Vernetzungsmoleküle ausgewählt werden.

Die Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metabolite und Abbauprodukte werden ausgewählt aus der folgenden Gruppe von Substanzen:

Polyacrylsäure und deren Derivate, Albumin, Metallchelatkomplexe, Cyclodextrine, Ionentauscher, lineare und cyclische Poly- und Oligoaminosäuren, modifizierte Polyaminosäuren, modifiziertes und nicht modifiziertes Polyethylenimin, Polyallylamin und modifiziertes Polyallylamin, basische Oligopeptide, immobilisierte Amidingruppen, Histidin, Polypropylen, Polyethylen, Polyvinylidenfluorid, Polytetrafluorethylen, Alkylarylgruppen, Monoaminoalkane, Toxisches Schocksyndrom Toxin 1 - bindende Peptide (toxic shock syndrome toxin 1 - bindende Peptide, TSST 1 - bindende Peptide), Diaminoalkane, Polyaminoalkane, aromatische stickstoffhaltige Heterozyklen und deren Derivate, antimikrobielle Peptide (AMP), Endotoxin-neutralisierendes-Protein (Endotoxin neutralizing protein, ENP), synthetische Peptide, Polylysin, HDL, Cholesterin, Polymyxin B und Polymyxin E (Colistin), membranbildende Lipide und Lipoproteine und Polysaccharide und Lipopolysaccharide, Glycoproteine, Cholesterinester, Triacylglycerine, allgemein Steroide, Phosphoglyceride, Sphingolipide, Lipoproteine mit und ohne cyclischen Anteil, Lipooligosaccharide mit Proteinanteil, Peptide mit der Formel R-(Lys-Phe-Leu)ₙ-R₁ mit R und R₁ = H oder worin R für eine Aminoschutzgruppe oder für Wasserstoff und R₁ für eine Carboxyschutzgruppe oder für Wasserstoff steht, Aminosäurereste, Fettsäurereste mit einer Länge zwischen 1-100 Kohlenstoffatomen, bevorzugt 1-10 Kohlenstoffatomen; stickstoffenthaltende heterocyclische Verbindungen, mit Stickstoffgruppen funktionalisierte aromatische Carbonsäuren und/oder deren Derivate. Heparin, Heparinderivate, Heparane, Heparanderivate, Oligosaccharide sowie Polysaccharide und vorzugsweise Oligosaccharide sowie Polysaccharide enthaltend Iduronsäure, Glucuronsäure, Glucosamin, Galactosamin sind erfindungsgemäß weniger oder nicht bevorzugt für die Endotoxinadsorption und werden daher nicht oder nicht bevorzugt zur Toxinadsorption eingesetzt.

Bevorzugte Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metabolite und Abbauprodukte sind Albumin, synthetische Peptide, Polylysin, Lipoproteine mit und ohne cyclischen Anteil, Lipooligosaccharide mit Proteinanteil, antimikrobielle Peptide (AMP), HDL, Cholesterin, Endotoxin-neutralisierendes-Protein und Toxisches Schocksyndrom Toxin 1 - bindende Peptide (toxic shock syndrome toxin 1 - bindende Peptide, TSST 1 - bindende Peptide).

Zum gezielten Beschichten der äußeren Oberfläche der Träger in Hohlfaserform bzw. zum gezielten Immobilisieren der Substanzen zur adsorptiven Entfernung von Toxinen biologischer und chemisch-synthetischer Herkunft, deren Metabolite und Abbauprodukte auf dieser Oberfläche werden zunächst die Poren und dann der Innenraum, das Lumen, der Träger mit einem Medium gefüllt. Das Medium ist unter den Bedingungen des Füllens flüssig und bedeckt daher vollständig die Oberfläche des Lumens sowie der Poren. Zudem ist dieses Medium nicht mischbar mit einer Lösung, die anschließend zur Beschichtung der äußeren Oberfläche der Träger in Hohlfaserform eingesetzt wird. Aufgrund der Tatsache, dass das Medium die Oberfläche des Lumens sowie die innere Oberfläche der Poren vollständig bedeckt und mit der Lösung zur Beschichtung der äußeren Oberflächen der Träger nicht mischbar ist, kann die Lösung zur Beschichtung der äußeren Oberflächen der Träger die Oberflächen des Lumens oder die inneren Oberflächen der Poren nicht beschichten, so dass eine Beschichtung nur auf den äußeren Oberflächen der Träger in Hohlfaserform stattfindet. Nach der Beschichtung der äußeren Oberflächen der Träger in Hohlfaserform, welche bevorzugt vollständig bzw. quantitativ abläuft, wird das Medium aus dem Lumen und den Poren des Trägers entfernt.

Zum gezielten Beschichten der Oberfläche des Lumens der Träger in Hohlfaserform werden zunächst die Poren mit dem Medium gefüllt. Dann wird das Lumen der Träger mit der Lösung gefüllt, die zur Beschichtung der Oberfläche des Lumens in Hohlfaserform eingesetzt wird. Nach der Beschichtung der Oberflächen des Lumens, welche bevorzugt vollständig bzw. quantitativ abläuft, wird das Medium aus den Poren des Trägers und die Lösung aus dem Lumen der Träger entfernt.

Ähnlich können die äußere Oberfläche und die Oberfläche des Lumens der Träger in Hohlfaserform beschichtet werden, in dem zunächst die Poren mit dem Medium gefüllt werden und dann das Lumen mit der Lösung zur Beschichtung gefüllt und die äußere Oberfläche der Träger mit der Lösung zur Beschichtung umgeben werden. Als Medium werden lineare verzweigte, acyclische oder cyclische C₁ bis C₂₀-Alkane, beispielsweise Hexan, Heptan oder Dodecanol verwendet.

Man erhält einen Träger in Hohlfaserform, der nur auf seinen äußeren Oberflächen eine Beschichtung aufweist, während die Oberflächen seines Lumens unbeschichtet bleiben. Oder man erhält einen Träger in Hohlfaserform, der nur auf den Oberflächen seines Lumens, während seine äußeren Oberflächen unbeschichtet bleiben. Durch das Befüllen des Lumens und der Poren der Trägers konnten somit gezielt bestimmte Oberflächen der Träger vor bestimmten Beschichtungen geschützt werden.

Zum Beschichten der äußeren Oberfläche der Träger in Form von Partikeln sowie der inneren Oberflächen seiner Poren bzw. zum Immobilisieren der Substanzen zur adsorptiven Entfernung von Toxinen biologischer und chemisch-synthetischer Herkunft, deren Metabolite und Abbauprodukte auf diesen Oberflächen werden die Partikel in der Lösung zur Beschichtung suspendiert und dabei auch die Poren gefüllt. Nach der Beschichtung der äußeren Oberflächen der Träger ein Partikelform sowie der inneren Oberflächen seiner Poren, welche bevorzugt vollständig bzw. quantitativ abläuft, wird die Lösung von den Partikeln und aus den Poren des Trägers entfernt.

Die erhaltenen Träger weisen aufgrund der gezielt beschichteten und unbeschichteten Oberflächen entsprechend unterschiedliche Eigenschaften auf diesen Oberflächen auf.

Bei den Trägern in Hohlfaserform wird entweder die äußere Oberfläche oder die Oberfläche des Lumens mit Substanzen beschichtet, so dass entweder die äußere Oberfläche oder die Oberfläche des Lumens von Blut, Blutersatzmitteln oder Lösungen zum Einbringen in den menschlichen und/oder tierischen Blutkreislauf umströmt werden kann. Die jeweils andere Oberfläche der Träger in Hohlfaserform bleibt unbeschichtet. Da die Träger bevorzugt aus hydrophoben Polymeren bestehen, weist die unbeschichtete Oberfläche also hydrophobe Eigenschaften auf. Über diese unbeschichtete Oberfläche wird der Gasstrom geleitet.

Damit liegen sich in einem Träger in Hohlfaserform die unbeschichtete Oberfläche, über die der Gasstrom geleitet wird, und die mit Substanzen beschichtete Oberfläche, die mit Blut, Blutersatzmitteln oder Lösungen zum Einbringen in den menschlichen und/oder tierischen Blutkreislauf in Kontakt steht, gegenüber. Beide Oberflächen sind verbunden über die Poren, die durch die Wand der Hohlfaserträger führen. Die Toxine biologischer und chemisch-synthetischer Herkunft, deren Metabolite und Abbauprodukte adsorbieren an den Substanzen, mit denen entweder das Lumen oder die äußere Oberfläche der Träger beschichtet sind und werden auf diesen Oberflächen zurückgehalten. Die Toxine biologischer und chemisch-synthetischer Herkunft, deren Metabolite und Abbauprodukte werden also aus dem Blut, den Blutersatzmitteln oder den Lösungen zum Einbringen in den menschlichen und/oder tierischen Blutkreislauf entfernt. Handelt es sich um Blut, diffundiert das darin enthaltene Kohlendioxid durch die Poren des Trägers hindurch in den gasdurchströmten Raum hinein und wird vom Gasstrom abtransportiert. Gleichzeitig wird der Blutstrom aber von dem geringen Durchmesser der Poren und von der hydrophoben Eigenschaft der mit dem Gasstrom in Kontakt stehenden Oberfläche und der inneren Oberfläche der Poren daran gehindert, ebenfalls durch die Poren in den gasdurchströmten Raum überzutreten.

Der Durchmesser der Poren wird so gewählt, dass er kleiner ist als der Durchmesser einer Blutzelle. Eine Porengröße von ≤ 1,5 µm ist bevorzugt, mehr bevorzugt ist eine Porengröße von ≤ 1,0 µm, da die maximale Porengröße von 1,5 µm kleiner ist als die kleinsten Blutzellen, welche einen Durchmesser von etwa 2 µm haben. Die Blutzellen können daher nicht in die Poren des Trägers eindringen.

Der in dem Gasstrom enthaltene Sauerstoff kann ebenfalls durch die Poren des Trägers diffundieren und so in den blutdurchströmten Raum gelangen. Dadurch kommt es zur Anreicherung des Bluts mit Sauerstoff. Auf diese Weise können also gleichzeitig aus dem Blut Toxine biologischer und chemisch-synthetischer Herkunft, deren Metabolite und Abbauprodukte und Kohlendioxid entfernt und Sauerstoff im Blut angereichert werden.

Wie oben angesprochen wird der Blutstrom u.a. von der hydrophoben Eigenschaft der mit dem Gasstrom in Kontakt stehenden Oberfläche der Träger in Hohlfaserform, und der inneren Oberfläche der Poren daran gehindert, durch die Poren des Trägers in den gasdurchströmten Raum überzutreten. In Geräten, welche nur einen Gasaustausch in Blut vornehmen, ist das Übertreten von z.B. Blutplasma in den gasdurchströmten Raum ein häufig auftretendes und ernsthaftes Problem, dass den Gasaustausch behindert. Für die erfindungsgemäße Vorrichtung mit Doppelfunktion hat sich im Vergleich zu einem Gerät, welches nur einen Gasaustausch in Blut durchführt, überraschenderweise herausgestellt, dass ein Übertreten von z.B. Blutplasma in den gasdurchströmten Raum nicht bzw. nur sehr selten auftritt und der Gasaustausch zuverlässig, ohne Behinderung und mit hohen Gastransferraten stattfindet.

Die innere Oberfläche oder die äußere Oberfläche der Träger in Form von Hohlfasern oder die äußere Oberfläche der Träger in Form von Partikeln können zusätzlich zu der Beschichtung mit Substanzen eine hemokompatible Beschichtung aufweisen. Die hemokompatible Beschichtung wird dabei jeweils auf die Seite der Hohlfasern aufgebracht, welche mit Blut, Blutersatzmitteln oder Lösungen zum Einbringen in den menschlichen und/oder tierischen Blutkreislauf in Kontakt kommen wird. Die hemokompatible Beschichtung verhindert oder verringert Reaktionen des Bluts auf die als fremd erkannten Oberflächen der Vorrichtung und führt daher zu schonendem Umgang mit dem Patienten. Überraschend hat sich herausgestellt, dass bei Beschichtung der Trägeroberflächen mit Substanzen und zusätzlich einer hemopompatiblen Beschichtung beide Beschichtungen ihre Funktionen in vollem Umfang und ohne sich gegenseitig zu behindern ausführen können. Dabei besteht die Funktion der Beschichtung der Trägeroberflächen mit Substanzen in der Entfernung von Toxinen biologischer und chemisch-synthetischer Herkunft, deren Metabolite und Abbauprodukte aus Blut, Blutersatzmitteln oder Lösungen zum Einbringen in den menschlichen und/oder tierischen Blutkreislauf. Die Funktion der hemokompatiblen Beschichtung besteht in der Verhinderung oder Verringerung von Reaktionen des Bluts auf die als fremd erkannten Oberflächen der Träger und der Vorrichtung. Es war befürchtet worden, dass die zusätzliche hemokompatible Beschichtung der Trägeroberflächen zu einer Veränderung von deren Oberflächeneigenschaften führen könnte, die sich nachteilig auf die Wechselwirkung der ebenfalls auf der Trägeroberfläche befindlichen Substanzen mit den Toxinen biologischer und chemisch-synthetischer Herkunft, deren Metabolite und Abbauprodukte auswirken könnte und so die Bindungskapazität der Substanzen für die genannten Toxine herabsetzen könnte. Diese Befürchtung hat sich überraschenderweise nicht bestätigt.

Die hemokompatible Beschichtung besteht aus Heparin oder aus chemisch modifizierten Polysacchariden, also aus chemischen Derivaten der Polysaccharide. Die chemischen Modifizierungen der Polysaccharide bestehen in Desulfatierung, Resulfatierung, Deacylierung und/oder Reacylierung in unterschiedlichen Ausmaßen. Die Polysaccharide werden ausgewählt aus der folgenden Gruppe:
Glycosaminoglycane, synthetische Oligo- und Polysaccharide, Glucosaminoglykane, chemisch modifiziertes Heparin und Heparan-Sulfate, Chondroitin-Sulfate, Dermatan-Sulfate, Keratan-Sulfate, Hyaluronane, Onuphinsäure, Carrageenane, Chitine, Xylane, Dextrane, Mannane, Xyloglucane, Galactane, Xanthane, Arabinogalacturonane, Rhamnogalacturonane, Galaktomanane, Pektine, Amylopektine, Lambda, Agar-Agar, Agarose, Algin, Alginate, Ghatti-Gummi, Gummi-Arabicum, Traganthe, Karaja-Gummi, Johannisbrotkernmehl, Guas-Gummi, Tara-Gummi, Manucol, Kelgine, Pululan, Isolichenin, Nigeran Mycodextran, Elsinoe Leucospila a-Glycan, Alternan, Evernia prunastri a-Glycan, Pustulan, Islandic acid, Luteic acid, Microellobosporia Mannoglucan, Agrobacterium b-Glucane, Rhizobium b-Glucane, Acetobacter b-Glucan, Mycoplasma b-Glucan, Escherichia coli (1-2)-b-Oligoglucoside, Curdlan, Laminarin, Paramylon, Chrysolaminarin, Cellulin, Mycolaminarin, Lichenin, Callose, Furcellaran, Heparin, Urokinase, HEMA-St-HEMA Copolymer und poly-HEMA sowie deren chemische Derivate.
Eine derartige hemokompatible Beschichtung ist optional und auch nur in wenigen Fällen bevorzugt, wobei die für die hemokompatible Beschichtung verwendeten Substanzen nicht für die Adsorption der Toxine eingesetzt werden und auch nicht zur Adsorption der Toxine beitragen.

Zusätzlich kann die innere Oberfläche oder die äußere Oberfläche der Hohlfasern eine Oberflächenspannung herabsetzende Beschichtung aufweisen. Die Oberflächenspannung herabsetzende Beschichtung wird dabei jeweils auf die Seite der Hohlfasern aufgebracht, welche mit Blut in Kontakt kommen wird. Die Herabsetzung der Oberflächenspannung führt zu einem effizienten Priming der Vorrichtung.

Vor der Anwendung der Vorrichtung an einem Patienten muss der gesamte für den Blutstrom vorgesehene Innenraum der Vorrichtung mit Flüssigkeit gefüllt werden. Dieser Vorgang wird als Priming bezeichnet und das dazu benötigte Flüssigkeitsvolumen als Prime Volume. Das Priming ist notwendig, um unerwünschtes Gas vollständig aus dem blutführenden Innenraum der Vorrichtung zu entfernen, die Oberflächen des blutführenden Raumes gut zu benetzen und beim Verbinden der Vorrichtung mit dem Blutkreislauf des Patienten einen komplett flüssigkeitsgefüllten extrakorporalen Blutkreislauf sicherzustellen. Unter dem "blutführenden Innenraum der Vorrichtung" oder dem "blutführenden Raum" werden alle Räume bzw. Oberflächen der Vorrichtung verstanden, die mit Blut, Blutersatzmitteln oder den Lösungen zum Einbringen in den menschlichen und/oder tierischen Blutkreislauf in Kontakt kommen. Zu dem blutführenden Innenraum der Vorrichtung oder dem blutführenden Raum gehört auch die Kammer der Vorrichtung, die jeweils von Blut, Blutersatzmitteln oder Lösungen zum Einbringen in den menschlichen und/oder tierischen Blutkreislauf durchströmt wird.

Eventuell auf die Oberflächen der blutführenden Räume der Vorrichtung aufgebrachte Benetzungsmittel können vorteilhafterweise während des Primings entfernt werden und unterstützen den Priming-Vorgang. Das Prime Volume ist dabei abhängig von dem Volumen der Gesamtheit der blutführenden Innenräume der Vorrichtung. Je größer das Volumen der blutführenden Innenräume ist, desto größer wird auch das Volumen der Priming-Flüssigkeit, welches sich über den extrakorporalen Kreislauf mit dem Blutkreislauf des Patienten mischt. Die Mischung der Priming-Flüssigkeit mit dem Blutkreislauf des Patienten führt zur Hemodilution, die für den Patienten eine zusätzliche Belastung bedeutet. Deshalb ist es vorteilhaft, das Prime Volume so gering wie möglich zu halten. Als Priming-Flüssigkeit können die folgenden Lösungen oder eine Mischung davon eingesetzt werden: Kochsalzlösung 0,9%, Ringer-Lactat-Lösung, HAES, Mannitol, Heparin, Cortison, Natriumbicarbonat-Lösung, Tranexamsäure (früher Aprotenin).

Zur Herabsetzung der Oberflächenspannung können die Oberflächen der Hohlfasern mit einem Benetzungsmittel beschichtet werden. Als Benetzungsmittel können amphotere, zwitterionische, nichtionische, anionische und/oder kationische Verbindungen verwendet werden. Die Benetzungsmittel für die Oberflächenspannung herabsetzende Beschichtung werden aus der Gruppe der folgenden Verbindungen ausgewählt.

Amphotere Benetzungsmittel umfassen z.B. Lauroamphocarboxyglycinat, z.B. MIRANOL 2MHT MOD erhältlich bei Miranol, Inc. (Dayton, New Jersey) oder synergistische Bestandteile davon. Beispielhafte zwitterionische Benetzungsmittel umfasssen B-N-Alkylaminopropionsäure, N-alkyl-B-Iminodipropionsäure, Fettsäure-Imidazolincarboxylate, N-Alkylbetaine, Sulfobetaine, Sultaine, und Aminosäuren, z.B. Asparagin, L-Glutamin usw. Beispiele für anionische Benetzungsmittel umfassen aromatische hydrophobe Ester und anionische fluorhaltige Benetzungsmittel. Zu den kationischen Benetzungsmitteln zählen methylbis-hydrogeniertes Talgamidoethyl, 2-Hydroxyethylammoniummethylsulfat, wasserlösliche quarternierte Kondensatpolymere, Cocoalkylbis-(2-hydroxyethyl)-methyl und ethoxylierte Chloride. Nichtionische Benetzungsmittel umfassen alkoxylierte Alkylamine, Ethanol, Isopropanol, Methanol, Glycerin, Alkylpyrrolidone, lineare Alkoholalkoxylate, fluorierte Alkylester einschließlich Aminoperfluoroalkylsulfonat, N-Alkylpyrrolidone, alkoxylierte Amine, und Poly(methylvinylether/maleinsäureanhydrid)-Derivative. Andere Benetzungsmittel umfassen oligomere oder nicht-monomere Verbindungen enthaltend C12-18 aliphatische und/oder aromatische hydrophobe Reste und eine hydrophile Funktionalität innerhalb des gleichen Moleküls. Weitere Benetzungsmittel umfassen difunktionale Block-Copolymere mit endständigen sekundären Hydroxylgruppen und difunktionale Block-Copolymere mit endständigen primären Hydroxylgruppen. Diese Block-Copolymere enthalten typischerweise Wiederholungsblöcke von Poly(oxypropylen) oder Propylenoxide (POP) und Poly(oxyethylen) oder Ethylenoxid (POE). Nichttoxische und hemokompatible Benetzungsmittel werden bevorzugt.

Die Oberflächenspannung herabsetzende Beschichtung mit Benetzungmittel wird reversibel auf die innere oder äußere Oberfläche der Hohlfasern aufgebracht, so dass das Benetzungsmittel von den Oberflächen heruntergewaschen werden kann, bevor die Oberflächen mit Blut in Kontakt treten.

Die erfindungsgemäßen Vorrichtungen werden zum Entfernen von Toxinen biologischer und chemisch-synthetischer Herkunft, deren Metabolite und Abbauprodukte aus Blut, Blutersatzmitteln oder Lösungen zum Einbringen in den menschlichen und/oder tierischen Blutkreislauf verwendet. Die Toxine biologischer und chemisch-synthetischer Herkunft, deren Metabolite und Abbauprodukte werden ausgewählt aus der Gruppe:
Fibrinogen, Toxine im Zusammenhang mit einer Infektionskrankheit, Toxine im Zusammenhang mit der Ernährung, z.B. Pilzgifte, Nikotin, Ethanol, Botulismus; Toxine aus beruflich bedingten und kriminellen Handlungen heraus, z.B. Bleiacetat, B- und C-Waffen; Toxine in Form von Gas, Aerosol, Flüssigkeit und Feststoff, z. B. CO; Immunkomplexe, Medikamente, Drogen, Alkohol, Detergentien, Phosgen, Chlor, Blausäure, Nitrosamine, Oxalsäure, Benzpyrene, Solanin, Nitrate, Nitrite, Amine, Dichlordisulfid, halogenierte Kohlenwasserstoffe; Toxine bakterieller, mykotischer, z.B. Mykotoxine wie Epoxytrichotecene, Ochratoxin A, Zearalenon; und protozoonaler Herkunft und deren Bestandteile, z.B. Exotoxine, Endotoxine, Pilzsporen; und deren Abbauprodukte, biologische Kampfgifte wie Microcystine.; Anatoxine, Saxitoxine bakterieller Herkunft und deren Abbauprodukte, Insektizide, Bakterizide, Drogen und deren Metaboliten, Rauschmittel, Arzneimittel und deren Metabolite und ihre Abbauprodukte, Antigene, DNA, RNA, ENA, Immunglobuline, Autoimmunantikörper, Antikörper, auch Anti-DNA-Antikörper, Anti Nuclear-Antikörper, Viren, Retroviren und Virenbestandteile, z.B. Hepatitisvirenpartikel; Lipide, Proteine, Peptide, Proteolipide, Glycoproteine und Proteoglycane, Fibrin, Prionen, Nanowaffen, Metalle; z. B. Hg, Cd, Pb, Cr, Co, Ni, Zn, Sn, Sb sowie Ionen dieser Metalle; Halbmetalle, z. B. As; sowie Ionen dieser Halbmetalle, toxische Lipopolysaccharide und Endotoxine.

Bevorzugte Toxine biologischer und chemisch-synthetischer Herkunft, deren Metabolite und Abbauprodukte sind toxische Lipopolysaccharide und Endotoxine.

Die Endotoxine oder toxischen Lipopolysaccharide können beispielsweise aus den folgenden Organismen stammen: Escherichia coli, Salmonella, Shigella, Pseudomonas, Neisseria, Haemophilus influenzae, Bordetella pertussis und Vibrio cholerae.

Chemisch entsprechen die Endotoxine Lipopolysacchariden (LPS); LPS sind amphipathische Moleküle. Der hydrophobe Teil, das Lipid A, enthält fünf bis sieben gesättigte Fettsäuren, die an ein Glucosamin-Dimer gebunden sind. Der hydrophile Kopf des LPS-Moleküls besteht aus einem Oligosaccharid, der zentralen Core-Region und dem O-Antigen, einem Polymer aus sich wiederholenden Einheiten von jeweils drei bis sechs auch innerhalb einer Bakterienmembran variierenden Zuckerresten (Neutralzucker mit 5-7 C-Atomen, Desoxy- und Aminozucker, Uron- und Aminouronsäuren, O-Methyl-, O-Acetyl-, Phosphat- und Aminosäuresubstituierte Zucker). Die Core-Region enthält viele negativ geladene Kohlenhydratreste und Phosphatreste und bindet außerdem divalente Kationen, so dass eine Art Permeabilitätsbarriere entsteht.

Die pathophysiologischen Interaktionen beruhen nach heutigem Wissensstand auf dem toxisch wirksamen Bestandteil des Endotoxins, dem Lipid A. Es reagiert mit Rezeptoren auf Zellen des Immunsystems, hauptsächlich Makrophagen. Lipid A bindet dabei zunächst an das membranständige CD 14 (Cluster of Differentiation). Durch einen bisher ungeklärten Mechanismus der intrazellulären Signaltransduktion produzieren und sezernieren die betroffenen Zellen daraufhin Entzündungsmediatoren (IL-1, IL-6, IL-12, TNF-α) und aktivieren damit das Immunsystem, einschließlich des humoralen Immunsystems.

Im Rahmen der Reaktion auf die Bindung des Lipid A werden von den Makrophagen auch CD 14 in die Umgebung freigesetzt. Diese können dann auch Zellen, die im Grunde nicht auf das Lipid A reagieren, beeinflussen. Beispielsweise exprimieren Zellen des Endothels nach Bindung von CD 14 vermehrt Selektine und Integrine, die wiederum eine vermehrte Adhäsion von Leukozyten und Thrombozyten an den Gefäßwänden bewirken.

Die vermehrte Adhäsion von Thrombozyten an die Gefäßwand führt zur Aktivierung der Blutgerinnung und Freisetzung von Kininen (z.B. Bradykinin) mit der Folge der Entstehung von Thromben, die im Zuge ihres Abbaus die Fibrinolyse anstoßen. Die Kininfreisetzung bewirkt zudem eine Vasodilatation.

Zusammengefasst lässt sich sagen, dass durch die Wirkungen des Endotoxins das Gleichgewicht zwischen Entzündungsgeschehen, Gerinnung und Lyse gestört ist. Mögliche Folgen bestehen in Entzündungen, vermittelt durch die Einwirkung von Mediatoren und durch Aktivierung des Komplementsystems. Schwerwiegende Folgen sind Organversagen, hervorgerufen durch Störungen der Mikrozirkulation bei Thrombusbildung und durch Schock infolge der Vasodilatation, sowie Verbrauchskoagulopathie durch Aktivierung von Blutgerinnung und Fibrinolyse.

Da die Core-Region viele negative Ladungen enthält, wechselwirkt sie bevorzugt mit elektrophilen oder positiv geladenen Gruppen, wie z.B. mit organischen Ammoniumionen. Daraus erklärt sich auch die Adsorption von LPS und Endotoxinen an stickstoffhaltige Verbindungen, wie antimikrobielle Peptide (AMP), Endotoxin-neutralisierendes-Protein (Endotoxin neutralizing protein, ENP), synthetische Peptide, Polymyxin B und Polymyxin E (Colistin), Albumin, Peptide mit der Formel R-(Lys-Phe-Leu)ₙ-R mit R und R₁ = H, Aminosäurerest, Fettsäurerest und n = 1-100 bevorzugt 1-10; Polyethylenimin, Polyaminosäuren, stickstoffenthaltende heterocyclische Verbindungen, mit Stickstoffgruppen funktionalisierte aromatische Carbonsäuren und/oder deren Derivate.

Die erfindungsgemäßen Vorrichtungen werden zum Anreichern von Blut, Blutersatzmitteln oder Lösungen zum Einbringen in den menschlichen und/oder tierischen Blutkreislauf mit Sauerstoff und zum Entfernen von Kohlendioxid aus Blut, Blutersatzmitteln oder Lösungen zum Einbringen in den menschlichen und/oder tierischen Blutkreislauf verwendet. Bei einer Fließgeschwindigkeit des Bluts, der Blutersatzmittel oder der Lösungen zum Einbringen in den menschlichen und/oder tierischen Blutkreislauf von 1 L/min erreichen die Vorrichtungen einen Sauerstofftransfer bis zu 100 ml/min und bei einer Fließgeschwindigkeit des Bluts, der Blutersatzmittel oder der Lösungen zum Einbringen in den menschlichen und/oder tierischen Blutkreislauf von 7 L/min einen Sauerstofftransfer bis zu 650 ml/min. Der Kohlendioxidtransfer beträgt bis 80 ml/min bei einer Fließgeschwindigkeit des Bluts, der Blutersatzmittel oder der Lösungen zum Einbringen in den menschlichen und/oder tierischen Blutkreislauf von 1 L/min und bis zu 350 ml/min bei einer Fließgeschwindigkeit des Bluts, der Blutersatzmittel oder der Lösungen zum Einbringen in den menschlichen und/oder tierischen Blutkreislauf von 7 L/min.

Die erfindungsgemäßen Vorrichtungen werden zum gleichzeitigen Entfernen von Toxinen biologischer und chemisch-synthetischer Herkunft, deren Metabolite und Abbauprodukte und Kohlendioxid aus Blut, Blutersatzmitteln oder Lösungen zum Einbringen in den menschlichen und/oder tierischen Blutkreislauf und zum Anreichern von Blut, Blutersatzmitteln oder Lösungen zum Einbringen in den menschlichen und/oder tierischen Blutkreislauf mit Sauerstoff verwendet. Das Entfernen von Toxinen biologischer und chemisch-synthetischer Herkunft, deren Metabolite und Abbauprodukte, z.B. Endotoxinen, und Kohlendioxid aus Blut und das Anreichern von Blut mit Sauerstoff haben sich gemeinsam als eine wirksame Vorbeugung, Linderung oder Behandlung von Erkrankungen erwiesen. Die erfindungsgemäßen Vorrichtungen werden also zur Vorbeugung, Linderung oder Behandlung von Erkrankungen verwendet, die durch die Toxine biologischer und chemisch-synthetischer Herkunft, deren Metabolite und Abbauprodukte verursacht werden.

Die erfindungsgemäßen Vorrichtungen haben sich weiterhin als wirkungsvoll erwiesen gegen Erkrankungen, die durch den Zerfall von gram-negativen Bakterien verursacht werden. Die erfindungsgemäßen Vorrichtungen werden also zur Vorbeugung, Linderung oder Behandlung von Erkrankungen angewendet, die auf die Anwesenheit von Lipopolysacchariden oder Endotoxinen als Membranbruchstücke gramnegativer Bakterien zurückzuführen sind.

Die Erkrankungen, die durch Toxine biologischer und chemisch-synthetischer Herkunft, deren Metaboliten und Abbauprodukte verursacht werden oder die auf die Anwesenheit von Lipopolysacchariden oder Endotoxinen als Membranbruchstücke gramnegativer Bakterien zurückzuführen sind, werden aus der folgenden Gruppe von Erkrankungen ausgewählt:

Endotoxinämie, Sepsis, Fieber, Entzündungen, Organversagen, Multiorganversagen, Verbrauchskoagulopathie, Rhabdomyolyse, Nekrosenbildung, Schock, Traumata, Bakteriämie, Diarrhoe, Leukozytose, Vasodilatation, Koagulation aufgrund von Hypotension, Kreislaufversagen, Systemisches inflammatorisches Reakfionssyndrom (Systemic Inflammatory Response syndrome = SIRS), Aternnotsyndrom des Erwachsenen (Acute Respiratory Distress Syndrome =ARDS), etc.

Insbesondere stellt die gemeinsame Anwendung der genannten Behandlungen eine effektive Möglichkeit zur Vorbeugung, Linderung oder Behandlung von Sepsis dar. Besonders vorteilhaft ist die gleichzeitige Anwendung der genannten Behandlungen, die durch die erfindungsgemäßen Vorrichtungen ermöglicht wird. Denn sie erspart dem durch die Sepsis stark geschwächten Patienten, zwei bzw. drei verschiedene Behandlungen zu verkraften. Sie ist also nicht nur sehr effektiv, sondern auch sehr schonend. Zudem geht mit der synchronen Behandlung keine kostbare Zeit mehr verloren, die durch eine getrennte Behandlung von Organersatztherapie und einer nachgestellten Endotoxinadsorption zwangsläufig entstehen würde. Für das Krankenfiauspersonal entsteht bei synchroner Anwendung keine zusätzliche Arbeitsleistung und ebenso wird die Entscheidung nicht mehr zu treffen sein, ob und wann eine Endotoxinadsorption angewendet werden soll. Diese Abnahme der Entscheidungsfindung sowie die sequentielle Anwendung zweier oder mehrerer Therapien während der kritischen Phase eines Patienten spart wertvolle Zeit, so dass das Risiko für den Patienten an der Sepsis zu sterben deutlich reduziert wird.

Die erfindungsgemäßen Vorrichtungen werden zur Anwendung eines Verfahrens zum Entfernen von Toxinen biologischer und chemisch-synthetischer Herkunft, deren Metabolite und Abbauprodukte aus Blut, Blutersatzmitteln oder Lösungen zum Einbringen in den menschlichen und/oder tierischen Blutkreislauf verwendet, welches die folgenden Schritte umfasst.
a) Bereitstellen einer Vorrichtung zum Entfernen von Toxinen biologischer und chemisch-synthetischer Herkunft, deren Metabolite und Anbauprodukte aus Blut, Blutersatzmitteln oder Lösungen zum Einbringen in den menschlichen und/oder tierischen Blutkreislauf; und
b) Durchleiten von Blut, Blutersatzmitteln oder Lösungen zum Einbringen in den menschlichen und/oder tierischen Blutkreislauf.

Dabei können die Säulen I und/oder II, welche in der erfindungsgemäßen Vorrichtung vorhanden sind, als Einmal-Artikel eingesetzt werden oder für eine weitere Anwendung regeneriert werden. Damit kann das Verfahren zum Entfernen von Toxinen biologischer und chemisch-synthetischer Herkunft, deren Metabolite und Abbauprodukte aus Blut, Blutersatzmitteln oder Lösungen zum Einbringen in den menschlichen und/oder tierischen Blutkreislauf einen zusätzlichen Schritt c) umfassen:
c) Regeneration der Vorrichtung.

Die erfindungsgemäßen Vorrichtungen werden weiter zur Anwendung eines Verfahrens zum Anreichern von Blut, Blutersatzmitteln oder Lösungen zum Einbringen in den menschlichen und/oder tierischen Blutkreislauf mit Sauerstoff und zum Entfernen von Kohlendioxid aus Blut, Blutersatzmitteln oder Lösungen zum Einbringen in den menschlichen und/oder tierischen Blutkreislauf verwendet, welches die folgenden Schritte umfasst.
a) Bereitstellen einer Vorrichtung zum Anreichern von Blut, Blutersatzmitteln oder Lösungen zum Einbringen in den menschlichen und/oder tierischen Blutkreislauf mit Sauerstoff und zum Entfernen von Kohlendioxid aus Blut, Blutersatzmitteln oder Lösungen zum Einbringen in den menschlichen und/oder tierischen Blutkreislauf;
b) Durchleiten von Blut, Blutersatzmitteln oder Lösungen zum Einbringen in den menschlichen und/oder tierischen Blutkreislauf; und gegebenenfalls
c) Regeneration der Vorrichtung.

Die erfindungsgemäßen Vorrichtungen werden zum gleichzeitigen Entfernen von Toxinen biologischer und chemisch-synthetischer Herkunft, deren Metabolite und Abbauprodukte und Kohlendioxid aus Blut, Blutersatzmitteln oder Lösungen zum Einbringen in den menschlichen und/oder tierischen Blutkreislauf und zum Anreichern von Blut, Blutersatzmitteln oder Lösungen zum Einbringen in den menschlichen und/oder tierischen Blutkreislauf mit Sauerstoff verwendet, welches die folgenden Schritte umfasst.
a) Bereitstellen einer Vorrichtung zum gleichzeitigen Entfernen von Toxinen biologischer und chemisch-synthetischer Herkunft, deren Metabolite und Abbauprodukte und Kohlendioxid aus Blut, Blutersatzmitteln oder Lösungen zum Einbringen in den menschlichen und/oder tierischen Blutkreislauf und zum Anreichern von Blut, Blutersatzmitteln oder Lösungen zum Einbringen in den menschlichen und/oder tierischen Blutkreislauf mit Sauerstoff;
b) Durchleiten von Blut, Blutersatzmitteln oder Lösungen zum Einbringen in den menschlichen und/oder tierischen Blutkreislauf; und gegebenenfalls
c) Regeneration der Vorrichtung.

Ein Verfahren zur Endotoxinentfernung besteht in einem extrakorporalen Verfahren. Zunächst wird eine der erfindungsgemäßen Vorrichtungen mit einer wässrigen Lösung benetzt, gegebenenfalls das Benetzungsmittel aus der Vorrichtung ausgewaschen und dann die mit einer für den Patienten verträglichen Lösung gefüllte Vorrichtung über Schläuche; mit dem Blutkreislauf des Patienten verbunden. Das Blut wird dem Patienten kontinuierlich oder diskontinuerlich (Single needle-Anwendung) entnommen, die Toxine biologischer und chemisch-synthetischer Herkunft, deren Metabolite und Abbauprodukte aus dem Blut in der Vorrichtung gebunden sowie gleichzeitig das Blut mit Sauerstoff angereichert. Das behandelte Blut wird dem Patienten kontinuierlich oder diskontinuierlich wieder zugeführt.

### Beispiele

### Beispiel 1 :

### Immobilisierung von Albumin auf der äusseren Hohlfaseroberfläche einer Vorrichtung mit Säule I

### 1) Aminierung der äusseren Hohlfaseroberfläche

Um zu verhindern, dass das Lumen und die Poren der Hohlfaser ebenfalls aminiert werden, wird die Säule **I** mit einem mit Wasser nicht mischbaren Lösungsmittel, in diesem Fall Dodecanol, befüllt und anschliessend dieses über die Ein- und Auslässe, die die äussere Oberfläche betreffen, abgelassen und vorsichtig mit isotonischer Kochsalzlösung und danach mit Wasser gewaschen. Das Lumen und die Poren der Hohlfaser bleiben mit dem Dodecanol gefüllt, so dass gewährleistet ist, dass lediglich die äussere Oberfläche der Hohlfaser im folgenden aminiert wird.

Die in Säule I befindlichen Cellulosehohlfasern werden mit einer Lösung aus 10%iger Polyethyleniminlösung 60 Minuten lang bei Raumtemperatur mit einer Geschwindigkeit von 1 ml/s derart durchspült, dass die Lösung über den Ein- und Auslass durch die Säule **I** geleitet wird, so dass nur die äussere Oberfläche der Hohlfasern benetzt wird. Dazu wird ein Verhältnis der Gewichte der Hohlfasern zu der Polyethyleniminlösung von 1 : 2 (w:w) eingestellt. Anschliessend wird mit isotonischer Kochsalzlösung und reichlich Wasser bis zur Neutralität gewaschen.

### 2) Immobilisierung von Albumin

Die Akitivierung der Carboxylgruppen von Albumin wird mit CME-CDI (N-Cyclohexyl-N'-(2-morpholinoethyl)-carbodiimid-methyl-p-toluolsulfat) vorgenornrnen. Hierzu wird eine Reaktionslösung aus Albumin und CME-CDI im Gewichtsverhälniss 1:1 (w/w) bei 4°C in 0,1 M MES-Puffer (2-(N-Morpholino)ethansulfonsäure) bei pH 4,75 hergestellt und für eine halbe Stunde gerührt.
Die Reaktionslösung wird 4 Stunden bei Raumtemperatur über die äussere Oberfläche der aminierten Hohlfasern geleitet. Anschließend wird mit PBS-Puffer und Wasser bis zur Neutralität gewaschen.

Das in den Poren und im Lumen vorteilhafterweise flüchtige befindliche Dodecanol wird über Luftstrom entfernt und die Säule **I** wird über Nacht bei RT getrocknet.

### Beispiel 2 :

### Immobilisierung von Polyaminosäuren oder Peptiden auf Polysulfon

Hohlfasern oder Partikel aus Polysulfon werden, wie in J Polym Sci Part A: Polym Chem 41: 1316-1329, 2003 beschrieben, durch Reaktion mit n-Butyllithium, anschliessend mit Benzonitril und Reduktion mit Cyanoborhydrid in saurem Medium zum Benzylamin, mit Aminogruppen versehen. Die anschliessende Immobilisierung von Polylysin wird, wie in Beispiel 1 beschrieben, über die Aktivierung der C-terminalen Aminosäure des Polylysins mit dem Carbodiimid CME-CDI und anschliessender Reaktion der funktionellen Gruppen zur Peptidbindung erreicht.

Auf die gleiche Weise wurden antimikrobielle Peptide (AMP) und HDL bzw. Cholesterin auf Hohlfasern oder Partikel aus Polysulfon gebunden.

### Beispiel 3 :

### Immobilisierung von Heparin auf Partikeln

100g Trägermaterial in Form von Partikeln aus Polymethacrylat werden mit 300 ml einer 25%igen (w/v) Ammoniaklösung 3h bei Raumtemperatur an einem Rotationsverdampfer (Verwendung von Rührer zerstört die Partikel) bei langsamen Umdrehungsbewegungen inkubiert. Danach wird die Reaktionslösung von den Partikeln abfiltriert und die aminierten Partikel bis zur Neutralität mit dest. Wasser gewaschen.

1,5 g Heparin wird in einer Lösung aus 220 ml einer 0,1 M MES-Pufferlösung und 7,5 g CME-CDI bei 4°C vollständig aufgelöst und für 30 min bei 4°C gerührt. Diese Lösung wird anschliessend zu den aminierten Partikeln gegeben und über Nacht bei 4°C im Kühlraum rotiert.

Nach dieser Zeit wird das nicht kovalent gebundene Heparin mit einer 4 M wässrigen NaCl-Lösung von den modifizierten Partikeln abgespült und die modifizierten Partikel danach 30 Minuten mit dest. Wasser gespült.

### Beispiel 4:

### Füllen der Poren von Partikel

Das Füllen der Poren von Partikeln mit Dodecanol verhindert die Immobilisierung von Substanzen auf der Porenoberfläche.

Dazu werden die Partikel in einen geeigneten Rundkolben gefüllt und soviel Dodecanol zugefügt, dass die Partikel vollständig von Dodecanol bedeckt sind. Nach 10 Minuten wird das Dodecanol abfiltriert. Die Poren bleiben hierbei mit Dodecanol gefüllt.

### Beispiel 5 :

### Immobilisierung von Heparin auf der äusseren und inneren Hohlfaseroberfläche

Zunächst werden die Poren der Hohlfaser (Polyethersulfon) mit Dodecanol gefüllt., indem eine Säule vollständig, also beide Kammern, mit Dodecanol gefüllt wird und nach ca. 10 Minuten geleert wird. Dabei bleiben die Poren mit Dodecanol gefüllt. Das Modul wird anschliessend auf 4°C gekühlt.

Zunächst erfolgt eine Aminierung der Hohlfaseroberflächen entsprechend Beipiel 1. Nachdem 7,5 mg CME-CDI in 220ml 0,1 M MES-Puffer pH 4,75 bei ebenfalls 4°C aufgelöst ist, wird die entstandene Lösung für 30 Minuten bei 4°C durch die Säule I gepumpt und danach entfernt. Nach Spülen mit 250 ml kaltem MES-Puffer wird über Nacht bei gleicher Temperatur die Immobilisierungslösung aus 1,5g Heparin in 275 ml MES-Puffer (pH 4,75) durch die Säule I gepumpt.

Am nächsten Tag wird das nicht kovalent gebundene Heparin mit 4M NaCl_{aq} und anschließend mit dest. Wasser für 30 Minuten aus der Säule I ausgespült. Die Entfernung von Dodecanol aus den Poren gelingt mit 40°C warmem Isopropanol. Die derart mit Heparin beschichteten Hohlfasern werden wieder mit Wasser, 4M NaCl_{aq} und wieder Wasser gespült und anschließend getrocknet.

### Beispiel 6 :

### Immobilisierung von Toxischem Schocksyndrom Toxin 1 - bindenden Peptiden (TSST 1 - bindende Peptide) auf Hohlfasern

Eine Vorrichtung mit Säule **I** mit der Porengröße der Hohlfasern von 0,65 µm, einem inneren Durchmesser der Hohlfasern von 0,5mm und einer Membranfläche von 0,14 m² wurde im Kreis mit einer Lösung von 94 mg FeSO₄ X 7 H₂O und 84 mg Na₂S₂O₅ in 200 ml Wasser durchspült. Nach 15 Minuten werden in das Vorratsgefäß der Lösung zuerst 3,4 ml Methacrylsäure und 2 Minuten danach 3,4 ml Wasserstoffperoxyd (30%ig) gegeben. Anschließend wird die Lösung noch 2 weitere Stunden im Kreis gepumpt. Danach wird die Vorrichtung mit Säule **I** zur Entfernung von Reagenzienresten gleichzeitig auf der Innen- und Außenseite der Hohlfasern 4 Stunden lang mit fließendem Wasser gespült. Die Vorrichtung mit Säule **I** wird danach vollständig entleert.

In 220 ml einer 0,1 m MES (2-(N-Morpholino)ethansulfonsäure) Pufferlösung (pH 4,75) werden 7,5g CME-CDI (N-Cyclohexyl-N'-(2-morpholinoethyl)carbodiimid-methyl-p-toluolsulfonat) bei 4°C vollständig aufgelöst. Diese Lösung wird bei 4°C 30 Minuten im Kreis durch die Vorrichtung mit Säule **I** gepumpt. Anschließend wird die Säule **I** leergepumpt und möglichst schnell mit 250 ml 4°C kaltem 0,1 M MES Puffer (pH4,75) gespült. Nach Entfernung der Spüllösung wird eine Lösung von 1 g TSST 1-bindendem Peptid (toxic shock syndrome toxin 1 - bindendes Peptid, Auftragssynthese bei Bachem, Sequenz: GADRSYLSFIHLYPELAGA) in 200 ml 0,1 M MES Puffer bei 4°C für 18 Stunden in der Vorrichtung mit Säule **I** im Kreis gepumpt. Danach wird die Säule **I** leergepumpt und mit Wasser, mit 4 M Kochsalzlösung und wieder mit Wasser gespült und im Vakuum vollständig, getrocknet.

Die getrocknete Vorrichtung mit Säule **I** wird vollständig mit Dodecanol gefüllt und nach 10 Minuten wieder vollständig geleert. Die Säule **I** wird auf 4°C gekühlt.

### Beispiel 7 :

### Entfernung des TSST 1-bindenden Peptides von der äußeren und luminalen Hohlfaseroberfläche;

Die gekühlte Vorrichtung mit Säule I, wie in Beispiel 6 hergestellt, wurde mit 4°C kalter 6 M Salzsäure in und um die Hohlfasern gefüllt und 15 Stunden bei 4°C gelagert. Anschließend wird die Säule **I** geleert und die Lösung von Bestandteilen des TSST 1 - bindenden Peptids in der 6 M Salzsäure aufgefangen. Danach wurde Säule **I** mit 4°C kaltem Wasser pH neutral gespült und anschließend mit 40°C warmem Isopropanol gespült. Danach wurde noch einmal mit Wasser, mit 4 M Kochsalzlösung und erneut mit Wasser gespült und getrocknet.

### Beispiel 8 :

### Beschichtung der inneren und äusseren Oberfläche von Polyetherimidhohlfasern mit Polyacrylsäure

### 1. Aminierung der Polyetherimidhohlfaseroberfläche

Zur Aminierung der Oberflächen der Hohlfasern werden beide Kammern einer Säule **I** über die Einlässe langsam mit einer 4%igen wässrigen (entgastes dest. Wasser) Diethylenaminlösung luftblasenfrei befüllt und für 30 Minuten auf 90°C erhitzt. Anschließend wird die derart aminierte Säule **I** mit viel warmem und danach kaltem dest. Wasser bis zur pH-Neutralität gewaschen und getrocknet.

### 2. Beschichtung mit Polyacrylsäure

Die Aktivierung der Carbonylgruppe der Polyacrylsäure wird mit EDC vorgenommen. Hierzu werden 25g einer 10%igen Polyacrylsäurelösung (w/w) in 175 g einer isotonoschen NaCl-Lösung gelöst und auf pH 4,75 eingestellt. Anschließend wird die Polyacrylsäure-Lösung mit einer Lösung aus 2,50 g von N-(3-Dimethylaminopropyl)-N-Ethylcarbodiimid Hydrochlorid (EDC) in 100 ml isotonischer NaCl-Lösung gemischt und für 45 Minuten bei Raumtemperatur gerührt.

Die aminierten Hohlfasern werden anschließend mit der aktivierten Polyacrylsäurelösung versetzt und für mindestens 4 Stunden bei Raumtemperatur mit den Hohlfasern inkubiert.

### Beispiel 9 :

### Beschichtung von Hohlfasern mit Endotoxin-neutralisierendem-Protein (ENP)

Hierzu wird ENP auf die äußere Oberfläche von Polypropylenhohlfasern in Säule I immobilisiert.

Hierzu wird 30 Minuten lang bei 40°C 200 ml eines Gemisches aus Ethanol/Wasser 1/1 (v/v) durch die erste Kammer in Säule **I** im Kreis gepumpt. Dann wurden 4 ml 3-(Triethoxysilyl)-propylamin zugegeben und weitere 15 Stunden bei 40°C im Kreis gepumpt. Danach wurde noch jeweils 2 Stunden mit 200 ml Ethanol/Wasser und 200 ml Wasser gespült.
220 mg des ENP wurden bei 4°C in 30 ml 0,1 M MES Puffer pH 4,75 gelöst und mit 30 mg CME-CDI versetzt. Diese Lösung wurde für 15 Stunden bei 4°C im Kreis durch die erste Kammer in Säule **I** gepumpt**.** Anschließend wurde mit Wasser, 4M NaCl-Lösung und Wasser für je 2 Stunden gespült und getrocknet.

Auf die gleiche Weise, wie in diesem Beispiel 9 beschrieben, wurden Polyesterholhfasern und Silikonhohlfasern in einer Säule **I** erfolgreich mit ENP beschichtet.

### Beispiel 10 :

### Analyse der modifizierten Oberflächen von Partikeln und Hohlfasern

Zur Bestimmung des Gehalts an immobilisierter Substanz auf dem beschichteten Material (Partikel oder Hohlfasern) wurden die oberflächenmodizierten Polymere mit 3M Salzsäure bei 100°C für 16h inkubiert. Nach Entfernung der Salzsäure wurde das Hydrolysat mittels Anionenaustauschchromatographie aufgetrennt.

Das Signal eines ausgewählten Bestandteiles der ehemals immobilisierten Substanz wird integriert und mit der Signalfläche des Hydrolysates eines Standardpräparates mit einer definierten Konzentration der ausgewählten Substanz verglichen. Der Gehalt der Proben der modifizierten Partikel oder Hohlfasern an immobilisierter Substanz wird aus dem Signalflächenverhältnis des Polymerhydrolysates und des Standardhydrolysates berechnet.

### Beispiel 11 :

### Oberflächenmodifizierung von Polypropylen-Hohlfasern

Die äussere Oberfläche von Polypropylen-Hohlfasern wird mit Albumin beschichtet. Zunächst wird das Fasermaterial und der Innenraum einer Säule mit Ethanol gereinigt.

Das Innere der Hohlfasern wird mit Dodecanol gefüllt. Die kovalente Anbindung von Albumin auf der äusseren Oberfläche der Polypropylen-Hohlfasern erfolgt nach einer angepassten Zwei-Schritt-Standardmethode.

Im ersten Schritt wird ein Oligomethacrylsäure-Spacer kovalent an das Polypropylen gebunden. Im nachfolgenden Schritt wird die Albuminbeschichtung an die Carboxylfunktion des Oligomethacrylsäure-Spacers gebunden. Es wurden 100 mg Albumin für die Beschichtung der Hohlfasern eingesetzt.

### Beispiel 12 :

### Oberflächenmodifizierung einer Säule I

Die Oberfläche der ersten Kammer einer Säule I wird mit Albumin beschichtet. Wie in Beispiel 11, wird zunächst die erste Kammer einer Säule I mit Ethanol gereinigt. Dann wird die erste Kammer der Säule I mit ihren Bluteinlass/-auslassanschlüssen an eine Schlauchpumpe angeschlossen. Zunächst werden 500 ml einer Lösung des Oligomethacrylsäure-Spacers im Kreislauf durch die erste Kammer von Säule I gepumpt. Danach werden 500 ml der Albumin-Lösung im Kreislauf durch die erste Kammer gepumpt. Anschließend wird die erste Kammer gründlich mit VE-Wasser gespült. Es wurden 220 mg Albumin eingesetzt.

Der Albumin-Gehalt der in Beispiel 11 beschichteten Pölypropylenhohlfasern und von Proben, die der in diesem Beispiel 12 beschichteten Säule I entnommen wurden, wurden bestimmt, wie in Beispiel 10 beschrieben. Die Ergebnisse sind in Tabelle 1 dargestellt.

**Tabelle 1: Vergleich der Albumin-Belegung auf Polypropylenfasern und der Oberfläche der ersten Kammer einer Säule I.**

| Tabelle 1 | Polypropylenfasern | Säule I |
|---|---|---|
| | | |
| Eingesetztes Polymer | 72 cm² | 5605 cm² |
| Volumen der Albuminlösung | 200 ml | 500 ml |
| Albuminmenge | 100 mg | 220 mg |
| Albuminbelegung | 32 pmol/cm² | 3,6 pmol/cm² |

Der gemessene Oberflächengehalt auf den Fasern (32 pmol/cm²) ist hoch genug, um eine tatsächliche zwölfmal so große Oberfläche zu beschichten als eine angenommen glatte Oberfläche. Damit ist die Beschichtung mit Albumin als vollständig und durchgängig auch für die Oberfläche der ersteh Kammer einer Säule I zu bewerten.

### Beispiel 13:

### Bestimmung des Thrombozytenverlustes an mit Albumin oberflächehmodifiziertem Polypropylen

Je 4 mL Polypropylen-Fasermaterial (PP) und mit Albumin oberflächenmodifiziertes PP-Fasermaterial (hergestellt wie in Beispiel 1 beschrieben) werden in je eine 5 mL-Infusionstropfkammer gegeben.

Die Einlässe der Tropfkammern werden mit einem Kunststoffdreiwegeventil verbunden. Das gesamte System wird mit 200 mL physiologischer Kochsalzlösung (NaCl) gespült. Bei einem Blutspender wird die untere Armvene mit einer Butterfly-Kanüle punktiert. Eine Blutprobe wird zur Bestimmung der Thrombozytenkonzentration abgenommen. Anschließend wird der Auslass der Butterfly-Kanüle mit einem weiteren Dreiwegehahn und mit dem noch freien Anschluß des Dreiwegehahnes mit den NaCl-gefüllten Tropfkammern verbunden. Man lässt im freien Fluß das Blut durch die beiden Tropfkammer laufen. Über den freien Anschluss des Dreiwegehahnes wird Heparin zur Antikoagulation zugegeben. Die Dosis an Heparin sollte so niedrig wie möglich sein und muss für jedes Experiment individuell festgelegt werden. Die zuerst auslaufende NaCl-Lösung wird verworfen. Das weiter mit mind. 3 mL/min fließende Blut wird aufgefangen, bis in den beiden Auffangbehältern unter den Tropfkammern etwa 50 mL Blut gesammelt sind. Von diesem Blut wird ebenfalls der Thrombozytengehalt bestimmt.

### Bestimmt werden

1. Die Thrombozytenwiederfindung in der Tropfkammer mit dem albuminbeschichteten Testmaterial
2. Die Thrombozytenwiederfindung in der Tropfkammer mit dem nicht oberflächenmodifizierten PP-Fasermaterial.
3. In einer Extramessung wird die Thrombozytenwiederfindung in einer Tropfkammer ohne Füllung (Referenzwert) bestimmt.

Die Thrombozytenwiederfindung beträgt für nicht modifiziertes Material ca. 52%, für albuminbeschichtetes Material ca. 56% und für die leere Tropfkammer ca. 84%.

### Beispiel 14:

### Oberflächenmodifizierung einer Säule I

Eine Säule **I** mit einem Polymethylpenten-Hohlfaserbündel wird mit ihren Bluteinlass/-auslassanschlüssen an eine Schlauchpumpe angeschlossen. Jeweils 500 ml der Polyethyleniminlösung und der Albumin / CME-CDI - Lösung (siehe Beispiel 1) werden im Kreislauf durch die Säule I gepumpt. Anschließend spült man gründlich mit VE-Wasser nach. Es wurden 220 mg Albumin eingesetzt.

Die Bestimmung des Albumin-Gehaltes erfolgt nach der Vorschrift in Beispiel 10.

### Beispiel 15:

### In vitro Untersuchung zur Adsorption von Endotoxinen an einer beschichteten Säule I

### Versuchsbedingungen:

Perfusat : mit Endotoxin (150 I.E., LPS von E. coli 055:B5, Sigma-Aldrich) versetztes bovines Citratplasma
   pH = 7,5; OFSP (Oberflächenspannung): 53,5 ± 0,8mN/m
Perfusatrate 10 ml/min
Gasrate 10 ml/min
Gastemperatur 22°C
Perfusattemperaturca. 37°C
Perfusionsdauer 2 Stunden

### Vorbereitende Massnahmen:

Das beschichtete Blutkompartiment von Säule I (erste Kammer) wurde mit CO₂ gespült, bis keine Luft mehr in den Kapillaren und im Blutraum war. Anschliessend wurde die Anlage (erste Kammer) mit einer isotonischen Kochsalzlösung bei angeschlossenem Blutwärmetauscher und Sauerstoffbegasung konditioniert.

### Versuch:

Die isotonische Kochsalzlösung wird kontinuierlich durch das mit Endotoxin versetzte Perfusat ersetzt. Nach einer Perfusionsdauer von 24h wird der Endotoxingehalt im Perfusat bzw. nach Plasmagewinnung mittels chromogenem Limulus Amöbozyten-Lysat-Test (LAL-Assay) bestimmt.

Auf die gleiche Weise, wie in diesem Beispiel 15 beschrieben, wurden Adsorptionsversuche von Endotoxinen aus bovinem Vollblut, physiologischer Kochsalzlösung sowie dem Blutersatzmittel Oxygent^{®} durchgeführt. Aus allen Lösungen wurde das Endotoxin zu etwa 90 % mit Hilfe der erfindungsgemäßen Vorrichtung mit Säule **I** entfernt.

### Beispiel 16:

Für Vorrichtungen mit Säule **II** wurden die gleichen Polymermaterialien für Hohlfasern oder Partikel eingesetzt wie für Säule I (Beispiele 1, 2, 3, 5, 6, 8, 11, 12) mit dem Unterschied, dass der Porendurchmesser größer gewählt wurde (0,01 bis 1,5 µm), um den Durchtritt von Filtrat durch die Wände von Hohlfasern in Säule **II** zu ermöglichen.
Die Beschichtungen der Hohlfasern oder Partikel in Säule **II** wurden mit den gleichen Substanzen vorgenommen und auf die gleiche Weise durchgeführt wie in Beispielen 1 - 6, 8, 9 und 12 beschrieben. Es ergaben sich die gleichen Gehalte an immobilisierter Substanz für die verschiedenen Polymermaterialien und deren Beschichtungen.

### Beispiel 17:

Für Vorrichtungen mit Säule **II** wurde ebenfalls die Adsorption von Endotoxinen an beschichteten Hohlfasern oder beschichteten Partikeln bestimmt. Die Adsorption wurde bestimmt wie in Beispiel 15 beschrieben. Für die Adsorption von Endotoxin aus bovinem Citratplasma, bovinem Vollblut, physiologischer Kochsalzlösung und aus dem Blutersatzmittel Oxygent^{®}, wurde ebenfalls eine Adsorption zu etwa 90 % mit Hilfe der erfindungsgemäßen Vorrichtung mit Säule **II** gefunden.

### Beispiel 18:

Für Vorrichtungen, in denen erfindungsgemäß Säule I mit Säule **II** kombiniert wurde, wurde ebenfalls die Adsorption von Endotoxinen an beschichteten Hohlfasern oder beschichteten Partikeln bestimmt. Dazu wurden die beiden Säulen hintereinander geschaltet, so dass die Endotoxin-enthaltende Lösung entweder zuerst durch Säule I und dann durch Säule II fließt oder die Endotoxin-enthaltende Lösung fließt in der umgekehrten Richtung durch die beiden Säulen. Die weiteren Bedingungen für die Durchführung der Adsorption sind identisch mit den in Beispiel 15 genannten. Für die Adsorption von Endotoxin aus bovinem Citratplasma, bovinem Vollblut, physiologischer Kochsalzlösung und aus dem Blutersatzmittel Oxygent^{®} wurden Adsorptionen zwischen 95% und 97% mit Hilfe der erfindungsgemäßen Vorrichtung bestehend aus Säule **I** und Säule **II** gefunden. Die Reihenfolge, in der die Säulen von der endotoxin-enthaltenden Lösung durchlaufen wurden, hatte auf das Ausmaß der Adsorption des Endotoxins keinen Einfluss.

### Beispiel 19:

Für Vorrichtungen, in denen erfindungsgemäß Säule **I** mit Säule **II** kombiniert wurde, wurde weiter die Gastransferrate für Sauerstoff und Kohlendioxid für Säule **I** sowie die Effektivität der Hämofiltration für Säule **II** bestimmt.

### Gastransfer, Säule I:

Zur Bestimmung des Gastransfers wurden jeweils vor dem Bluteinlass und nach dem Blutauslass von Säule I Sensoren für Sauerstoff und Kohlendioxid angebracht. Zusätzlich wurde der Gaseinlass von Säule I mit einer Sauerstoffquelle versehen. Bovines Citratplasma und bovines Vollblut wurden mit einer definierten geringen Menge Sauerstoff und einer definierten hohen Menge Kohlendioxid versetzt. In zwei aufeinanderfolgenden Versuchen wurde die Änderung des Sauerstoff-Partialdrucks und die Änderung des Kohlendioxid-Partialdrucks in dem begasten Citratplasma und Vollblut beim Zirkulieren durch die erste Kammer einer Säule I Gemessen. Die beiden Experimente mit Citratplasma und Vollblut wurden in einer Säule I mit unbeschichteten Hohlfasern aus Polymethylpenten durchgeführt und zum Vergleich in einer Säule **I** mit Polymethylpenten-Hohlfasern, welche mit ENP beschichtet waren. Der Vergleich zwischen den Säulen ergab für Citratplasma wie für Vollblut einen Sauerstofftransfer in Blut, der für die Säule mit beschichteten Hohlfasern etwa 20 % niedriger lag als für die Säule mit unbeschichteten Hohlfasern. Ähnlich verhielt es sich mit dem Transfer von Kohlendioxid aus dem Blut heraus, der für die Säule mit beschichteten Hohlfasern etwa 20 % niedriger lag als für die Säule mit unbeschichteten Hohlfasern.

### Hämofiltration, Säule II:

Die Effektivität der Hämofiltration für Säule II wurde bestimmt, indem die Parameter Kreatinin, Harnstoff und die Elektrolyte Natrium und Kalium jeweils vor und nach dem Zirkulieren von bovinem Citratplasma oder bovinem Vollblut gemessen wurden. Dazu wurde eine Säule II, welche Polyethersulfon-Hohlfasern enthielt, die mit Albumin beschichtet waren, eingesetzt. Die zweite Kammer dieser Säule wurde mit einem Auslass für Ultrafiltrat versehen und in den Kreislauf der ersten Kammer, durch welchen die zu filtrierende Flüssigkeit fließt, wurde eine Zuführung für Substitutionslösung angeschlossen. Die Konzentrationen von Kreatinin, Harnstoff und Natrium und Kalium wurden in den zu filtrierenden Flüssigkeiten, bovinem Citratplasma und bovinem Vollblut, folgendermaßen eingestellt:

| | |
|---|---|
| Kreatinin | 3 x 10⁻² mg/ml |
| Harnstoff | 2 mg/ml |
| Natriumionen | 250 mM |
| Kaliumionen | 10 mM |

In zwei aufeinanderfolgenden Experimenten zirkulierten jeweils 500 ml der zu filtrierenden Flüssigkeiten mit einer Flussrate von 200 ml/min für zwei Stunden durch den Kreislauf der ersten Kammer einer Säule II. Nach dieser Zeit wurde der Gehalt der oben aufgeführten Substanzen bestimmt. In Tabelle 2 ist der aus den zu filtrierenden Flüssigkeiten entfernte Anteil der aufgeführten Substanzen zusammengestellt.

| Tabelle 2 | Entfernter Anteil |
|---|---|
| | |
| Kreatinin | 67 % |
| Harnstoff | 70 % |
| Natriumionen | 61 % |
| Kaliumionen | 56 % |

Damit liegen die Konzentrationen aller Parameter nach der Filtration mit Säule II im normalen physiologischen Bereich.

### Beispiel 20:

Für Vorrichtungen mit Säule I wurden Hohlfasern mit kombinierten Beschickungen aus jeweils einer hemokompatiblen Substanz und einer toxin-bindenden Substanz versehen.

### 20A. Heparin und ENP auf Polymethytpenten-Hohlfasern:

Zunächst wurde die äussere Oberfläche von Polymethylpenten-Hohlfasern aminiert, wie in Beipiel 1 beschrieben. Nachdem 5 mg CME-CDI in 220ml 0,1 M MES-Puffer pH 4,75 bei 4°C aufgelöst ist, wird die entstandene Lösung für 30 Minuten bei 4°C durch die erste Kammer von Säule **I** gepumpt und danach entfernt. Nach Spülen mit 250 ml kaltem MES-Puffer wird über Nacht bei gleicher Temperatur die Immobilisierungslösung aus 1g Heparin in 275 ml MES-Puffer (pH 4,75) durch die erste Kammer von Säule **I** gepumpt. Am nächsten Tag wird das nicht kovalent gebundene Heparin mit 4M NaCl_{aq} aus der Kammer gespült und anschließend die Kammer mit dest. Wasser bis zur pH-Neutralität gewaschen. Als letzter Schritt wurde die Beschichtung mit ENP durchgeführt, wie in Beispiel 9 beschrieben.

### 20B. Heparin und Albumin auf Polymethacrylat-Hohlfasern:

Zur Aminierung der äusseren Oberfläche der Hohlfasern wurden 300 ml einer 25%igen (w/v) Ammoniaklösung für 3h bei Raumtemperatur durch die erste Kammer von Säule I geleitet, die die Polymethacrylat-Hohlfasern enthielt. Danach wurde die erste Kammer und damit auch die äussere Oberfläche der Hohlfasern mit dest. Wasser bis zur Neutralität gewaschen. Für den ersten Beschichtungsschritt wurde 1g Heparin in einer Lösung aus 220 ml einer 0,1 M MES-Pufferlösung und 5 g CME-CDI bei 4°C vollständig aufgelöst und für 30 min bei 4°C gerührt. Diese Lösung wurde anschliessend über Nacht bei gleicher Temperatur durch die erste Kammer von Säule **I** gepumpt. Nach dieser Zeit wurde, wie in Beispiel 20A. beschrieben, das nicht kovalent gebundene Heparin aus der ersten Kammer und der Oberfläche der Hohlfasern entfernt. Als letzter Schritt wurde die Immobilisierung von Albumin auf der äusseren Oberfläche der Holhfasern durchgeführt, wie in Beispiel 1 beschrieben.

### Beispiel 21:

Wie in Beispiel 15 angegeben, wurden auch die mit einer kombinierten Beschichtung versehenen Säulen I aus Beispielen 20A. und 20B, auf ihre Bindungskapazität für Endotoxine getestet. Gleichzeitig wurde der Transfer von Sauerstoff und Kohlendioxid für die Säulen I aus Beispielen 20A. und 20B bestimmt, wie in Beispiel 19 beschrieben.

Für die Adsorption von Endotoxin aus bovinem Citratplasma, bovinem Vollblut, physiologischer Kochsalzlösung und aus dem Blutersatzmittel Oxygent^{®} wurden Adsorptionen von etwa 90 % mit Hilfe der erfindungsgemäßen Vorrichtung bestehend aus Säule I des Beispiels 20A. oder des Beispiels 20B gefunden.

Der Transfer von Sauerstoff und Kohlendioxid für die Säulen I aus Beispielen 20A. und 20B lag im gleichen Bereich wie für die Säulen I in Beispiel 19 gemessen.

### Beispiel 22:

Die Hohlfasern in Säulen II wurden mit der gleichen kombinierten Beschichtung versehen, wie in Beispiel 20 für die entsprechenden Säulen I beschrieben. Die Effektivität der Hämofiltration für die Säulen II mit einer kombinierten Beschichtung wurde dann bestimmt, wie in Beispiel 19 angegeben.

Die Effektivität der Hämofiltration für die Säulen II mit einer kombinierten Beschichtung lag im gleichen Bereich wie für die Säulen I in Beispiel 19 gemessen.

## Patentansprüche

1. Eine Vorrichtung zur Reinigung von Blut, Blutersatzmitteln oder Lösungen zum Einbringen in den menschlichen und/oder tierischen Blutkreislauf und zum Gasaustausch in Blut, Blutersatzmitteln oder Lösungen zum Einbringen in den menschlichen und/oder tierischen Blutkreislauf umfassend eine Säule mit:
a) einem Einlass und einem Auslass für Gase oder Gasgemische,
b) einem Einlass und einem Auslass für Blut, Blutersatzmittel oder Lösungen zum Einbringen in den menschlichen und/oder tierischen Blutkreislauf,
c) mindestens einer gasdurchlässigen Membran und
d) einem Träger, der mit Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metaboliten und Abbauprodukte beschichtet ist.

2. Die Vorrichtung gemäß Anspruch 1 umfassend eine weitere Säule mit:
a) einem Auslass für Filtrats,
b) einem Einlass und einem Auslass für Blut, Blutersatzmittel oder Lösungen zum Einbringen in den menschlichen und/oder tierischen Blutkreislauf,
c) mindestens einer semipermeablen Membran, und
d) einem Träger, der mit Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metaboliten und Abbauprodukte beschichtet ist.

3. Die Vorrichtung gemäß Anspruch 1, wobei die gasdurchlässige Membran und der beschichtete Träger zu einer Einheit zusammengefasst sind.

4. Die Vorrichtung gemäß Anspruch 1 oder 3, wobei die gasdurchlässige Membran durchlässig ist für Sauerstoff und Kohlendioxid.

5. Die Vorrichtung gemäß Anspruch 1 - 4, wobei die gasdurchlässigen Membran nicht durchlässig ist für Flüssigkeiten und/oder wobei die gasdurchlässige Membran aus einem oder mehreren Bündeln Hohlfasern besteht.

6. Die Vorrichtung gemäß Anspruch 1 - 5, wobei die Hohlfasern der Membran Poren enthalten mit einem Durchmesser im Bereich von 0.01 - 5 µm und bevorzugt einen Durchmesser von 0.01 - 1,5 µm und/oder wobei die Hohlfasern der Membran einen Außendurchmesser von etwa 0,1 - 1,5 mm, einen Innendurchmesser von etwa 0,1 - 1 mm und eine Wandstärke von 5 - 200 µm, vorzugsweise 15 - 50 µm aufweisen.

7. Die Vorrichtung gemäß Anspruch 1 - 6, wobei der Träger in Form von Partikeln oder in Form von Hohlfasern vorliegt.

8. Die Vorrichtung gemäß Anspruch 5, 6 oder 7, wobei der Träger in Form von Partikeln vorliegt und/oder die Hohlfasern aus einem Material oder Polymer bestehen ausgewählt aus der Gruppe und/oder die Partikel aus einem Polymer bestehen, ausgewählt aus der Gruppe:
Silica, Silikone, Polyolefine, Polytetrafluorethylen, Polyesterurethane, Polyetherurethane, Polyurethane, Polyethylenterephthalate, Polymethylpentan, Polymethylpenten, Polysaccharide, Polypeptide, Polyethylene, Polyester, Polystyrole, Polyolefine, Polysulfonate, Polypropylen, Polyethersulfone, Polypyrrole, Polyvinylpyrrolidone, Polysulfone, Polymilchsäure, Polyglycolsäure, Polyorthoester, polyaromatische Polyamide, Aluminiumoxid, Gläser, Sepharose, Kohlenhydrate, Copolymere von Acrylaten oder Methacrylaten und Polyamiden; Polyacrylsäureester, Polymethacrylsäureester, Polyacrylsäureamide, Polymethacrylsäureamide, Polymethacrylat, Polyetherimid, Polyacrylnitril, Copolymere aus Ethylenglycoldiacrylat oder Ethylenglycoldimethacrylat und Glycidylacrylat oder Glycidylmethacrylat und/oder Allylglycidylether, regenerierte Cellulose, Celluloseacetat, hydrophobe Polymere unter Zusatz von hydrophilen Polymeren, Derivate und Copolymere der genannten Polymere.

9. Die Vorrichtung gemäß Anspruch 1 - 8, wobei der Träger in Form von Partikeln vorliegt und die Partikel einen Durchmesser von 50 µm - 5 mm aufweisen oder der Träger in Form von Partikeln vorliegt und Poren enthält mit einem Durchmesser im Bereich von 0.01 - 5 µm und bevorzugt einem Durchmesser von 0.01 - 1,5 µm.

10. Die Vorrichtung gemäß Anspruch 1 - 9, wobei die innere Oberfläche und/oder die äußere Oberfläche der Träger in Form von Hohlfasern und die innere und/oder äußere Oberfläche der Träger in Form von Partikeln beschichtet sind mit Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metaboliten und Abbauprodukte.

11. Die Vorrichtung gemäß Anspruch 1 - 10, wobei die Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metaboliten und Abbauprodukte direkt über die chemischen funktionellen Gruppen oder über Linker an die Oberflächen der Träger gebunden sind.

12. Die Vorrichtung gemäß Anspruch 1 - 11, wobei die Substanzen zur adsorptiven Entfernung von in Blut, Blutersatzmitteln oder Lösungen zum Einbringen in den menschlichen und/oder tierischen Blutkreislauf befindlichen Toxinen biologischer und chemisch-synthetischer Herkunft, deren Metaboliten und Abbauprodukte ausgewählt werden aus der Gruppe Polyacrylsäure, Derivate der Polyacrylsäure, Albumin, Metallchelatkomplexe, Cyclodextrine, Ionentauscher, Polyaminosäuren, modifizierte Polyaminosäuren, modifiziertes und nicht modifiziertes Polyethylenen, Polyallylamin, und modifiziertes Polyallylamin, basische Oligopeptide, immobilisierte Amidingruppen, Histidin, Polypropylen, Polyethylen, Polyvinylidenfluorid, Polytetrafluorethylen, Alkylarylgruppen, Monoaminoalkane, Toxisches Schocksyndrom Toxin 1 - bindende Peptide, Diaminoalkane, Polyaminpalkane, aromatische stickstoffhaltige Heterozyklen und deren Derivate, antimikrobielle Peptide, Endotoxin-neutralisierendes-Protein, synthetische Peptide, Polylysin, HDL, Cholesterin, Polymyxin B, Polymyxin E, Peptide mit der Formel R-(Lys-Phe-Leu)ₙ-R₁ mit R und R₁ = H, Aminosäurereste, membranbildende Lipide, membranbildende Lipoproteine, membranbildende Polysaccharide, membranbildende Lipopolysaccharide, Glycoproteine, Cholesterinester, Triacylglycerine, Steroide, Phosphoglyceride, Sphingolipide, Lipoproteine mit cyclischem Anteil, Lipoproteine ohne cyclischen Anteil, Lipooligosaccharide mit Proteinanteil, Fettsäurereste mit einer Länge zwischen 1-100 Kohlenstoffatomen, bevorzugt 1-10 Kohlenstoffatomen; stickstoffenthaltende heterocyclische Verbindungen, mit Stickstoffgruppen funktionalisierte aromatische Carbonsäuren und/oder deren Derivate.

## Claims

1. A device for the purification of blood, blood substitutes or solutions for the introduction into the human and/or animal blood circulation and for gas exchange in blood, blood substitutes or solutions for the introduction into the human and/or animal blood circulation comprising a column with:
a) an inlet and an outlet for gases or gas mixtures,
b) an inlet and an outlet for blood, blood substitutes or solutions for the introduction into the human and/or animal blood circulation,
c) at least one gas-permeable membrane and
d) a carrier, which is coated with substances for the adsorptive removal of toxins of biological and chemical synthetic origin, their metabolites and degradation products present in blood, blood substitutes or solutions for the introduction into the human and/or animal blood circulation.

2. The device according to claim 1 comprising an additional column with:
a) an outlet for filtrate,
b) an inlet and an outlet for blood, blood substitutes or solutions for the introduction into the human and/or animal blood circulation,
c) at least one semipermeable membrane, and
d) a carrier, which is coated with substances for the adsorptive removal of toxins of biological and chemical synthetic origin, their metabolites and degradation products present in blood, blood substitutes or solutions for the introduction into the human and/or animal blood circulation.

3. The device according to claim 1, wherein the gas-permeable membrane and the coated carrier are combined into one unit.

4. The device according to claim 1 or 3, wherein the gas-permeable membrane is permeable for oxygen and carbon dioxide.

5. The device according to claim 1 - 4, wherein the gas-permeable membrane is not permeable for liquids and/or wherein the gas-permeable membrane consists of one or more bundles of hollow fibers.

6. The device according to claim 1 - 5, wherein the hollow fibers of the membrane contain pores with a diameter in the range of 0.01 - 5 µm, and preferably a diameter of 0.01 - 1.5 µm and/or wherein the hollow fibers of the membrane have an outer diameter of about 0.1 - 1.5 mm, an inner diameter of about 0.1 - 1 mm and a wall thickness of 5 - 200 µm, preferably 15 - 50 µm.

7. The device according to claim 1 - 6, wherein the carrier is present in form of particles or in form of hollow fibers.

8. The device according to claim 5, 6 or 7, wherein carrier is present in form of particles and/or the hollow fibers consist of a material or polymer selected from the group of and/or the particles consist of a polymer selected from the group of:
silica, silicones, polyolefins, polytetrafluoroethylene, polyesterurethanes, polyetheruretanes, polyurethanes, polyethylene terephthalates, polymethylpentane, polymethylpentene, polysaccharides, polypeptides, polyethylenes, polyesters, polystyrenes, polyolefins, polysulfonates, polypropylene, polyethersulfones, polypyrroles, polyvinylpyrrolidones, polysulfones, polylactic acid, polyglycolic acid, polyorthoesters, polyaromatic polyamides, aluminum oxide, glasses, sepharose, carbohydrates, copolymers of acrylates or methacrylates and polyamides; polyacrylic acid ester, polymethacrylic acid ester, polyacrylamides, polymethacrylamides, polymethacrylate, polyetherimide, polyacrylonitrile, copolymers of ethylene glycol diacrylate or ethylene glycol dimethacrylate and glycidyl acrylate or glycidyl methacrylate and/or allyl glycidylether, regenerated cellulose, cellulose acetate, hydrophobic polymers with the addition of hydrophilic polymers, derivatives and copolymers of the aforementioned polymers.

9. The device according to claim 1 - 8, wherein the carrier is present in form of particles and the particles have a diameter of 50 µm - 5 mm or the carrier is present in form of particles and contains pores with a diameter in the range of 0.01 - 5 µm and preferably a diameter of 0.01 - 1.5 µm.

10. The device according to claim 1 - 9, wherein the inner surface and/or the outer surface of the carriers in form of hollow fibers and the inner and/or outer surface of the carriers in form of particles are coated with substances for the adsorptive removal of toxins of biological and chemical synthetic origin, their metabolites and degradation products present in blood, blood substitutes or solutions for the introduction into the human and/or animal blood circulation.

11. The device according to claim 1 - 10, wherein the substances for the adsorptive removal of toxins of biological and chemical synthetic origin, their metabolites and degradation products present in blood, blood substitutes or solutions for the introduction into the human and/or animal blood circulation are bound directly via chemical functional groups or via linkers to the surfaces of the carriers.

12. The device according to claim 1 - 11, wherein the substances for the adsorptive removal of toxins of biological and chemical synthetic origin, their metabolites and degradation products present in blood, blood substitutes or solutions for the introduction into the human and/or animal blood circulation are selected from the group of polyacrylic acid, derivatives of polyacrylic acid, albumin, metal chelate complexes, cyclodextrins, ion exchangers, polyamino acids, modified polyamino acids, modified and unmodified polyethylenimine, polyallylamine and modified polyallylamine, alkaline oligopeptides, immobilized amidine groups, histidine, polypropylene, polyethylene, polyvinylidene fluoride, polytetrafluoroethylene, alkylaryl groups, monoaminoalkanes, toxic shock syndrome toxin 1 - binding peptides, diaminoalkanes, polyaminoalkanes, aromatic nitrogen-containing heterocyclic compounds and their derivatives, antimicrobial peptides, endotoxin-neutralizing protein, synthetic peptides, polylysine, HDL, cholesterol, polymyxin B, polymyxin E, peptides having the formula R-(Lys-Phe-Leu)ₙ-R₁ with R and R₁ = H, amino acid residues, membrane-forming lipids, membrane-forming lipoproteins, membrane-forming polysaccharides, membrane forming lipopolysaccharides, glycoproteins, cholesterol esters, triacylglycerols, steroids, phosphoglycerides, sphingolipids, lipoproteins with cyclic portion, lipoproteins without cyclic portion, lipooligosaccharides with protein portion, fatty acid residues having a length between 1-100 carbon atoms, preferably 1-10 carbon atoms; nitrogen-containing heterocyclic compounds, nitrogen group-functionalized aromatic carboxylic acids and / or their derivatives.

## Revendications

1. Un dispositif pour la purification du sang, des produits de substitution du sang ou des solutions à introduire dans la circulation sanguine humaine et/ou animale et pour l'échange de gaz dans le sang, les produits de substitution du sang ou les solutions à introduire dans la circulation sanguine humaine et/ou animale comprenant une colonne avec:
a) une entrée et une sortie pour les gaz ou les mélanges gazeux,
b) une entrée et une sortie pour le sang, les produits de substitution du sang ou les solutions à introduire dans la circulation sanguine humaine et/ou animale,
c) au moins une membrane perméable au gaz, et
d) un support, qui est revêtu avec des substances pour l'enlèvement par adsorption des toxines d'origine biologique et chimio-synthétique, leurs métabolites et leurs produits de dégradation du sang, des produits de substitution à partir du sang ou des solutions à introduire dans la circulation sanguine humaine et/ou animale.

2. Le dispositif selon la revendication 1 comprenant une colonne supplémentaire avec:
a) une sortie pour le filtrat,
b) une entrée et une sortie pour le sang, les produits de substitution du sang ou les solutions à introduire dans la circulation sanguine humaine et/ou animale,
c) au moins une membrane semi-perméable au gaz, et
d) un support, qui est revêtu avec des substances pour l'enlèvement par adsorption des toxines d'origine biologique et chimio-synthétique, leurs métabolites et leurs produits de dégradation à partir du sang, des produits de substitution du sang ou des solutions à introduire dans la circulation sanguine humaine et/ou animale.

3. Le dispositif selon la revendication 1, où la membrane perméable au gaz et le support revêtu sont réunis dans une unité.

4. Le dispositif selon la revendication 1 ou 3, où la membrane perméable au gaz est perméable à l'oxygène et au dioxyde de carbone.

5. Le dispositif selon une quelconque des revendications 1 - 4, où la membrane perméable au gaz n'est pas perméable aux liquides et/ou la membrane perméable au gaz consiste en un ou plusieurs faisceau(x) de fibres creux.

6. Le dispositif selon une quelconque des revendications 1 - 5, où les fibres creux de la membrane contiennent des pores ayant un diamètre dans la gamme 0.01 - 5 µm et préférablement un diamètre de 0.01 à 1.5 µm et/où les fibres creux de la membrane ont un diamètre extérieur d'environ 0.1 - 1.5 mm, un diamètre intérieur d'environ 0.1 - 1 mm et une épaisseur de la paroi de 5 - 200 µm, préférablement de 15 - 50 µm.

7. Le dispositif selon une quelconque des revendications 1 - 6, où le support est présent sous forme de particules ou de fibres creux.

8. Le dispositif selon la revendication 5, 6 ou 7, où le support est présent sous forme de particules et/ou de fibres creux, qui consistent en un matériel et/ou un polymère sélectionné parmi le groupe et/ou les particules consistent en un polymère sélectionné parmi le groupe:
silice, silicones, polyoléfines, polytétrafluoroéthylène, polyesteruréthanes, polyétheruréthanes, polyuréthanes, polyéthylène téréphthalates, polyméthylpentane, polyméthylpentène, polysaccharides, polypeptides, polyéthylènes, polyesters, polystyrènes, polysulfonates, polypropylène, polyéthersulfones, polypyrroles, polyvinylpyrrolidones, polysulfones, acide polylactique, acide polyglycolique, polyorthoester, polyamides polyaromatiques, oxyde d'aluminium, verres, sépharose, carbohydrates, copolymères d'acrylate ou de méthacrylate et polyamide, polyacrylates, polyméthacrylates, polyacrylamides, polyméthacrylamides, polyméthacrylate, polyétherimide, polyacrylonitrile, co-polymères d'éthylèneglycoldiacrylate ou d'éthylèneglycoldiméthacrylate et glycidylacrylate ou glycidylméthacrylate et/ou allylglycidyléther, cellulose régénérée, acétate de cellulose, polymères hydrophobes avec addition de polymères hydrophiles, dérivés et co-polymères des polymères sous-mentionnés.

9. Le dispositif selon une quelconque des revendications 1 - 8, où le support est présent sous forme de particules et les particules ont un diamètre de 50 µm - 5 mm ou le support est présent sous forme de particules et contient des pores avec un diamètre dans la gamme de 0.01 - 5 µm et préférablement un diamètre de 0.01 - 1.5 µm.

10. Le dispositif selon une quelconque des revendications 1 - 9, où la surface intérieure et/ou la surface extérieure des supports sous forme de fibres creux et la surface intérieure et/ou la surface extérieure des supports sous forme de particules sont revêtues avec des substances pour l'enlèvement par adsorption des toxines d'origine biologique et chimio-synthétique, leurs métabolites et leurs produits de dégradation à partir du sang, des produits de substitution du sang ou des solutions à introduire dans la circulation sanguine humaine et/ou animale.

11. Le dispositif selon une quelconque des revendications 1 - 10, où les substances pour l'enlèvement par adsorption des toxines d'origine biologique et chimio-synthétique, leurs métabolites et leurs produits de dégradation à partir du sang, des produits de substitution du sang ou des solutions à introduire dans la circulation sanguine humaine et/ou animale sont liées directement via des groupements chimiques fonctionnelles ou via des linkers aux surfaces des supports.

12. Le dispositif selon une quelconque des revendications 1 - 11, où les substances pour l'enlèvement par adsorption des toxines d'origine biologique et chimio-synthétique, leurs métabolites et leurs produits de dégradation à partir du sang, des produits de substitution du sang ou des solutions à introduire dans la circulation sanguine humaine et/ou animale sont sélectionnées parmi le groupe: acide polyacrylique, dérivés d'acide polyacrylique, albumine, complexes métalliques chélatés, cyclodextrines, échangeurs ioniques, poly(amino acides), poly(amino acides) modifiés, polyéthylènimine modifiée et non-modifiée, polyallylamine et polyallylamine modifiée, oligopeptides basiques, groupements amidine immobilisés, histidine, polypropylène, polyéthylène, fluorure de polyvinylidène, polytétrafluoroéthylène, groupements alkylaryle, monoaminoalcanes, peptides de liaison à la toxine 1 du syndrome du choc toxique, diaminoalcanes, polyaminoalcanes, hétérocycles azotés aromatiques et leurs dérivés, peptides antimicrobiens, protéine neutralisant l'endotoxine, peptides synthétiques, polylysine, HDL, cholestérol, polymixine B, polymixine E, peptide de formule R-(Lys-Phe-Leu)ₙ-R₁ avec R et R¹ étant H, résidus d'acides aminés, lipides de formation de membrane, lipoprotéines de formation de membrane, polysaccharides de formation de membrane, lipopolysaccharides de formation de membrane, glycoprotéines, esters de cholestérol, triacylglycérine, stéroïdes, phosphoglycérides, sphingolipides, lipoprotéines avec portion cyclique, lipoprotéines sans portion cyclique, lipooligosaccharides avec partie protéique, résidus d'acides gras avec une longueur de 1 - 100 atomes de carbone, préférablement de 1 - 10 atomes de carbone, composés hétérocycliques azotés, acides carboxyliques aromatiques fonctionnalisés avec des groupements azotés et/ou leurs dérivés.
